# EUROPEAN PATENT SPECIFICATION

(11) **EP 3 988 523 B1**
(45) Date of publication and mention of the grant of the patent: **18.01.2023**
(21) Application number: 20382918.9
(22) Date of filing: 21.10.2020
(51) Int. Cl.: C07C 29/159, C07C 45/00, C07C 51/15, B01J 20/04

(54) **PROCESS FOR PRODUCING FUNCTIONALIZED ORGANIC MOLECULES**
VERFAHREN ZUR HERSTELLUNG VON FUNKTIONALISIERTEN ORGANISCHEN MOLEKÜLEN
PROCÉDÉ DE PRODUCTION DE MOLÉCULES ORGANIQUES FONCTIONNALISÉES

(43) Date of publication of application: 27.04.2022
(73) Proprietor: B. Braun Surgical, S.A., 08191 Rubi (Barcelona) (ES); Universitat Politècnica de Catalunya, 08034 Barcelona (ES)
(72) Inventor: TURÓN DOLS, Pau, 08191 Rubí (Barcelona) (ES); SANZ BELTRÁN, Vanesa, 08191 Rubí (Barcelona) (ES); RODRÍGUEZ RIVERO, Anna Maria, 08191 Rubí (Barcelona) (ES); ALEMÁN LLANSÓ, Carlos Enrique, 08019 Barcelona (ES); PUIGGALÍ BELLALTA, Jordi, 08019 Barcelona (ES); REVILLA-LÒPEZ, Guillem, 08019 Barcelona (ES); SANS, Jordi, 08019 Barcelona (ES)
(74) Representative: Patentanwälte Ruff, Wilhelm, Beier, Dauster & Partner mbB

(56) References cited:
- WO-A1-2017/086817
- WO-A1-2018/024727
- CN-A- 106 542 964
- US-A1- 2011 132 770
- DONG DONG ZHU ET AL: "Recent Advances in Inorganic Heterogeneous Electrocatalysts for Reduction of Carbon Dioxide", ADVANCED MATERIALS, vol. 28, no. 18, 1 May 2016 (2016-05-01), pages 3423-3452, XP055581016, ISSN: 0935-9648, DOI: 10.1002/adma.201504766

## Description

### FIELD OF THE INVENTION

The present invention, which is defined in the claims, relates to a process for producing functionalized organic molecules having 1 to 3 carbon atoms and to uses of this process.

### BACKGROUND OF THE INVENTION

Carbon dioxide (CO₂) is considered the primary greenhouse gas and the main cause for global climate warming. Therefore, the efficient utilization of it as C1 feedstock to synthesize valuable chemical and industrial products is drawing increasing attention. For example, it is known that carbon dioxide may be employed as C1 feedstock to synthesize ethanol (W. Zhang, Y. Hu, L. Ma, G. Zhu, Y. Wang, X. Xue, R. Chen, S. Yang. Z. Jin, Adv. Sci. 2018, 5, 1700275; B. An, Z. Li, Y. Song, J. Z. Zhang, L. Z. Zeng, C. Wang, W. B. Lin, Natur. Catal. 2019, 2, 709-717; C. Liu, B. C. Colon, M. Ciesack, P. A. Silver, D. G. Nocera, Science 2016, 352, 1210-1213; E. S. Wiedner, J. C. Linehan, Chem. Eur. J. 2018, 24, 16964-16971; D. Wang, Q. Y. Bi, G. H. Yin, W. L. Zhao, F. Q. Huang, X. M. Xie, M. H. Jiang, Chem. Commun. 2016, 52, 14226-14229). Further, it is known that transition metals and complexes that can act as transition metals dominate the catalysis associated to CO₂ activation and fixation (C. S. Yeung, Angew. Chem. Int. Ed. 2019, 58, 5492-5502; C. Weetman, S. Inoue, ChemCatChem 2018, 10, 4213-4228; P. P. Power, Nature 2010 463 171-177; D. D. Zhu, J. L. Liu, S. Z. Adv. Mater. 2016, 28, 3423-3452).

Further, it is known that metals and transition-metal compounds (such as transition-metal oxides and transition-metal chalcogenides) and carbon-based materials may be used as inorganic heterogeneous electrocatalysts for reduction of carbon dioxide (Dong Dong Zhu et al.: "Recent Advances in Inorganic Heterogeneous Electrocatalysts for Reduction of Carbon Dioxide", Adv. Mater. 2016, 28, 3423-3452).

Since the carbon atom in CO₂ is at the highest oxidation state, the CO₂ molecule is very inert and stable. Therefore, the conversion of CO₂ into high-value chemicals with one carbon atom (C1; as for example methanol and formic acid), two carbon atoms (C2; as for example ethanol and acetic acid) and three carbon atoms (C3; as for example acetone) requires very efficient electrocatalysts for promoting the kinetically sluggish CO₂ reduction process.

Accordingly, there is still further need for processes facilitating the conversion of CO₂ into high-value chemicals, in particular as mentioned above.

### OBJECT AND SOLUTION

In view of the foregoing, the object underlying the present invention is therefore to make available a process for producing, in particular selectively producing, functionalized organic molecules having 1 to 3 carbon atoms, which addresses the afore-mentioned need.

This object is accomplished by a process according to independent claim 1 and by the uses according to the claims 15 and 16. Preferred embodiments of the process are defined in the dependent claims and the present description. The subject-matter and wording, respectively of all claims is hereby incorporated into the description by explicit reference.

The present invention relates to a process for producing or synthesizing, in particular selectively producing or synthesizing, functionalized organic molecules having 1 to 3 carbon atoms, wherein the functionalized organic molecules are selected from the group consisting of ethanol, methanol, formic acid, acetic acid, malonic acid, acetone and a mixture of at least two of the afore-said functionalized organic molecules. Preferably, the present invention relates to a process for producing or synthesizing, in particular selectively producing or synthesizing, ethanol or a mixture comprising or consisting of ethanol and at least one further functionalized organic molecule, preferably selected from the group consisting of methanol, formic acid, acetic acid, malonic acid and acetone, in particular to a mixture comprising or consisting of ethanol, methanol, formic acid, acetic acid and acetone, in particular with ethanol as a major reaction product, or to a mixture comprising or consisting of ethanol, methanol, acetic acid, malonic acid and acetone, in particular with ethanol as a major reaction product.

The process comprises the step of
- contacting a gas mixture comprising or consisting of carbon dioxide (CO₂) and methane (CH₄), in particular only comprising or consisting of carbon dioxide (CO₂) and methane (CH₄), in presence of water, in particular liquid water, (H₂O) with a catalyst, in particular electrocatalyst, comprising or consisting of permanently polarized hydroxyapatite.

Hereafter, the above step of the inventive process is denoted as "contacting step".

The term "functionalized organic molecules" as used according to the present invention means organic molecules bearing or comprising functional groups, i.e. specific substituents or moieties that are typically responsible for the characteristic chemical reactions of the organic molecules. The functional groups are selected from the group consisting of carboxy groups, keto groups and hydroxy groups.

Further, the term "functionalized organic molecules" as used according to the present invention may refer to one type of organic molecule, for example ethanol or formic acid, or to a mixture comprising or consisting of different organic molecules. The different organic molecules, for example, may be different in terms of the number of carbon atoms and/or the functional group.

The term "functionalized organic molecules" as used according to the present invention means carboxylic acids, ketones, alcohols or mixtures thereof. The carboxylic acids/carboxylic acid are/is formic acid and/or acetic acid and/or malonic acid. The ketones/ketone are/is acetone. The alcohols/alcohol are/is ethanol and/or methanol.

The process according to the present invention is a process for producing or synthesizing, in particular selectively producing or synthesizing, functionalized organic molecules which are selected from the group consisting of ethanol, methanol, formic acid, acetic acid, malonic acid, acetone and mixtures thereof, i.e. mixtures of at least two of the afore-said functionalized organic molecules.

Especially preferably, the process according to the present invention is a process for producing or synthesizing, in particular selectively producing or synthesizing, ethanol or a mixture comprising or consisting of ethanol, methanol, formic acid, acetic acid and acetone, in particular with ethanol as a major reaction product, or a mixture comprising or consisting of ethanol, methanol, acetic acid, malonic acid and acetone, in particular with ethanol as a major reaction product.

The term "major reaction product", in particular in the context of ethanol, as used according to the present invention means a product having the highest molar yield within a mixture comprising or consisting of different products, in particular different functionalized organic molecules, in particular having 1 to 3 carbon atoms.

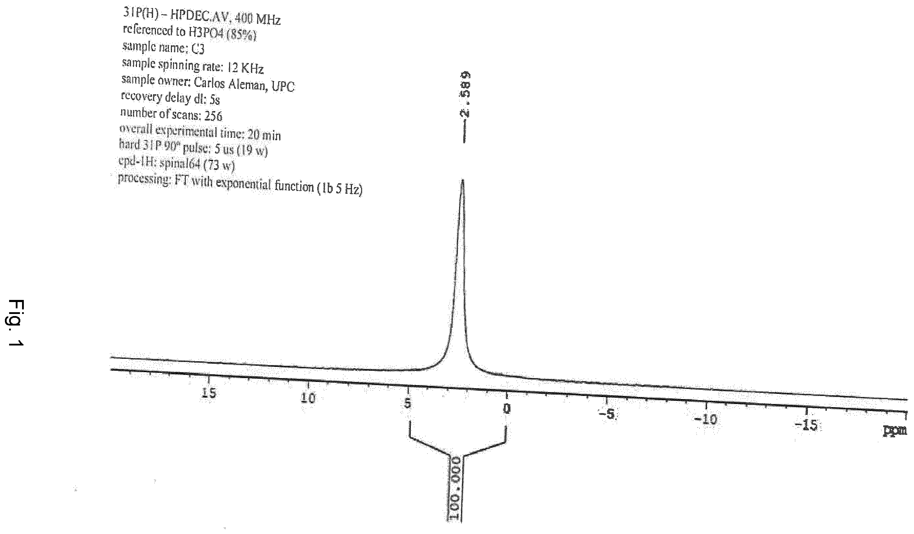

The term "permanently polarized hydroxyapatite" as used according to the present invention means a hydroxyapatite that has undergone a complete structural redistribution, in particular almost perfect, with a high crystallinity degree, i.e. particularly with a low amount of amorphous calcium phosphate and the presence of vacancies detected by increased electrochemical activity and the accumulation of charge per unit mass and surface. It has an electrochemical activity and ionic mobility which do not disappear over time. The corresponding ³¹P-NMR spectrum of the permanently polarized hydroxyapatite is as shown on fig. 1. Preferably, said spectrum is carried out with solid hydroxyapatite using phosphoric acid (H₃PO₄) as a reference and showing a unique peak at 2.6 ppm corresponding to phosphate groups of hydroxyapatite.

The term "thermally polarized hydroxyapatite" as used according to the present invention preferably means a permanently polarized hydroxyapatite obtained or obtainable by a process (thermal polarization process) comprising the steps of
(a) sintering samples of hydroxyapatite, in particular at a temperature between 700°C and 1200°C, and
(b) applying a constant or variable DC voltage between 250 V and 2500 V, in particular for at least 1 min and/or at a temperature between 900 °C and 1200 °C, in particular from 1000 °C to 1200 °C, or
   applying an equivalent field between 1.49 kV/cm and 15 kV/cm, in particular for at least 1 min and/or at a temperature between 900 °C and 1200 °C, in particular from 1000 °C to 1200 °C, or
   applying an electrostatic discharge between 2500 V and 1500000 V, in particular for > 0 min to 24 h, for example for less than 10 min, and/or at a temperature between 900 °C and 1200 °C, in particular from 1000 °C to 1200 °C, or
   applying an equivalent electrical field between 148.9 kV/cm and 8928 kV/cm, in particular for > 0 min to 24 h, for example for less than 10 min, and/or at a temperature between 900 °C and 1200 °C, in particular from 1000 °C to 1200 °C.

The samples of hydroxyapatite in step (a) may be samples of a natural, i.e. naturally occurring, hydroxyapatite or of a synthetic hydroxyapatite.

Further, the samples of hydroxyapatite in step (a) may be in particular selected from the group consisting of samples of crystalline hydroxyapatite, samples of amorphous hydroxyapatite, samples of a mixture of crystalline hydroxyapatite and amorphous calcium phosphate, and mixtures thereof.

Accordingly, the permanently polarized hydroxyapatite of the composition or material according to the present invention is preferably obtained or obtainable by the above process (thermal polarization process).

The term "room temperature" as used according to the present invention means a temperature from 15 °C to 35 °C, in particular 18 °C to 30 °C, preferably 20 °C to 30 °C, more preferably 20 °C to 28 °C, particularly 20 °C to 25 °C.

The present invention rests on the surprising finding that production or synthesis, in particular selective production or synthesis, of functionalized organic molecules having 1 carbon atom (methanol and/or formic acid), functionalized organic molecules having 2 carbon atoms (ethanol and/or acetic acid) and functionalized organic molecules having 3 carbon atoms (such as acetone) from carbon dioxide and methane in the presence of permanently polarized hydroxyapatite as catalyst is achievable under mild conditions (particularly < 10 bar pressure and ≤ 250 °C, in particular < 250 °C, temperature) with lower levels of environmental contamination and cost. Without wishing to be bound by theory, the production or synthesis of functionalized organic molecules having 1 to 3 carbon atoms according to the present invention involves hydrogenation of reduced carbon dioxide and C-C bond construction. Thus, the process according to the present invention may also be denoted as electro-reduction process of carbon dioxide towards carboxylic acids (such as formic acid and/or acetic acid) and/or ketones (acetone) and/or alcohols ( methanol and/or ethanol) and the permanently polarized hydroxyapatite may also be denoted as electro-catalyst.

In an embodiment of the invention, the permanently polarized hydroxyapatite comprises or has
- a crystallinity > 65 %, in particular > 70 %, preferably > 75 %, more preferably from 65 % to 99.9 %,
   and/or
- a proportion of amorphous calcium phosphate < 18 % by weight, in particular from 0.1 % by weight to 17 % by weight or < 9 % by weight, preferably < 5 % by weight, in particular < 0.1 % by weight, based on the total weight of the permanently polarized hydroxyapatite,
   and/or
- a proportion of β-tricalcium phosphate < 36 % by weight, in particular from 0.1 % by weight to 35 % by weight or < 12 % by weight, preferably < 5 % by weight, in particular < 0.5 % by weight, based on the total weight of the permanently polarized hydroxyapatite,
   and/or
- a bulk resistance from 10⁷ Ω cm² to 10⁴ Ω cm², in particular 10⁷ Ω cm² to 10⁵ Q cm², in particular 10⁶ Ω cm² to 10⁵ Ω cm², preferably of 10⁵ Q cm²,
   and/or
- a surface capacitance which decreases less than 8%, in particular from 8 % to 0.1 %, preferably from 5 % to 3 %, after 3 months.

The term "bulk resistance" as used according to the present invention means resistance to the electron transfer and may be determined by means of electrochemical impedance spectroscopy.

Preferably, the bulk resistance increases by only 0.1 % to 33 %, in particular 4 % to 63 %, preferably by 4 %, after 3 months.

The term "surface capacitance" as used according to the present invention means capacitance attributed to surface changes of hydroxyapatite induced by a thermal polarization process and may be determined by means of electrochemical impedance spectroscopy.

With respect to further features and advantages of the permanently polarized hydroxyapatite as used according to the present invention, it is referred to the PCT application WO 2018/024727 A1. In a further embodiment of the invention, the permanently polarized hydroxyapatite is obtained or obtainable by a process comprising the steps of
(a) preparing samples of hydroxyapatite, in particular crystalline hydroxyapatite,
(b) sintering the samples prepared in step (a), in particular at a temperature between 700 °C and 1200 °C,
(c) applying a constant or variable DC voltage between 250 V and 2500 V, in particular for at least 1 min and/or at a temperature between 900 °C and 1200 °C, in particular from 1000 °C to 1200 °C, to the samples obtained in step (b) or to shaped bodies thereof or
   applying an equivalent electric field between 1.49 kV/cm and 15 kV/cm, in particular for at least 1 min and/or at a temperature between 900 °C and 1200 °C, in particular from 1000 °C to 1200 °C, to the samples obtained in step (b) or to shaped bodies thereof or
   applying an electrostatic discharge between 2500 V and 1500000 V, in particular for > 0 min to 24 h, for example for less than 10 min, and/or at a temperature between 900 °C and 1200 °C, in particular from 1000 °C to 1200 °C, to the samples obtained in step (b) or to shaped bodies thereof or
   applying an equivalent electrical field between 148.9 kV/cm and 8928 kV/cm, in particular for > 0 min to 24 h, for example for less than 10 min, and/or at a temperature between 900 °C and 1200 °C, in particular from 1000 °C to 1200 °C, to the samples obtained in step (b) or to shaped bodies thereof and
(d) cooling the samples obtained in step (c) maintaining the DC voltage or the equivalent electric field, or
   cooling the samples obtained in step (c) maintaining the electrostatic discharge or the equivalent electric field, or
   cooling the samples obtained in step (c) without maintaining the DC voltage or electrostatic discharge.

The term "samples" as used according to the present invention may in particular mean one sample, i.e. only one sample (singular), or a plurality of samples, i.e. two or more samples. Accordingly, the term "shaped bodies" as used according to the present invention may in particular mean one shaped body, i.e. only one shaped body (singular), or a plurality of shaped bodies, i.e. two or more shaped bodies.

The aforementioned step (a) may be carried out by using ammonium phosphate dibasic (diammonium hydrogen phosphate, (NH₄)₂HPO₄) and calcium nitrate (Ca(NO₃)₂) as reactants or starting materials. In particular, the step (a) may be carried out by
(a₁) providing a mixture, in particular an aqueous mixture, preferably an aqueous-alcoholic mixture, of ammonium phosphate dibasic and calcium nitrate,
(a₂) stirring the mixture provided in step (a₁), in particular at room temperature,
(a₃) hydrothermal treating of the mixture stirred in step (a₂),
(a₄) cooling the mixture hydrothermally treated in step (a₃),
(a₅) separating precipitates obtained after cooling the mixture in step (a₄), and
(a₆) freeze-drying the precipitates separated in step (a₅) to produce hydroxyapatite, in particular crystalline hydroxyapatite.

The step (a₁) may be in particular carried out by using a mixture comprising or consisting of ammonium phosphate dibasic, calcium nitrate, water, in particular de-ionized water, ethanol, and optionally chelated calcium solutions. Advantageously, the pH value of the mixture and/or the pH value of an aqueous calcium nitrate solution applied for providing the mixture may be adjusted to 10-12, preferably 10.5. Thus, shapes and sizes of hydroxyapatite, in particular in the form of nanoparticles, can be controlled. Further, the step (a₂) may be carried out under agitation, in particular gentle agitation, for example applying 150 rpm to 400 rpm. Further, the step (a₂) may be carried out for 1 min to 12 h, in particular for 1 h. The step (a₂) may also be termed as an aging step, according to the present invention. Further, the step (a₃) may be carried out at a temperature of 60 °C to 240 °C, preferably of 150 °C. Further, the step (a₃) may be carried out at a pressure of 1 bar to 250 bar, preferably of 20 bar. Further, the step (a₃) may be carried out for 0.1 h to 72 h, preferably for 24 h. Further, the step (a₄) may be carried out by cooling the mixture hydrothermally treated in step (a₃) to a temperature of 0 °C to 90 °C, in particular of 25 °C. Further, the step (a₅) may be carried out by means of centrifugation and/or filtration. Further, the precipitates separated in step (a₅) may be washed, in particular using water and/or a mixture of ethanol and water, before the step (a₆) is carried out. Further, the step (a₆) may be carried out for 1 day to 4 days, in particular for 2 days to 3 days, preferably for 3 days.

Further, the aforementioned step (b) may be carried out at a temperature between 700 °C and 1150 °C, in particular between 800 °C and 1100 °C, in particular at 1000 °C.

Further, the process preferably comprises between the step (b) and the step (c) a further step (bc)
- pressing the samples obtained in step (b) to form shaped bodies or to form the shaped bodies thereof, i.e. to form the shaped bodies of the samples obtained in step (b).

In particular, the step (bc) may be carried out under a pressure of 1 MPa to 1000 MPa, in particular 100 MPa to 800 MPa, preferably 600 MPa to 700 MPa. Further, the step (bc) may be carried out for 1 min to 90 min, in particular 5 min to 50 min, preferably 10 min to 30 min.

The shaped bodies may have a polygonal, for example triangular, quadratic or rectangular, pentagonal, hexagonal, heptagonal, octagonal or nonagonal, or a corner-less, in particular circular, oval-shaped or elliptical, cross-section. Further, the shaped bodies may have a thickness of > 0 cm to 10 cm, in particular > 0 cm to 5 cm, preferably > 0 cm to 2 cm. In particular, the shaped bodies may have a thickness of 0.1 cm to 10 cm in particular 0.1 cm to 5 cm, preferably 0.5 cm to 2 cm.

Preferably, the shaped bodies are in the form of discs, plates, cones or cylinders.

Advantageously, by carrying out step (c), catalytic activation of the samples obtained in step (b) or the shaped bodies thereof may be accomplished. Preferably, step (c) is carried out by placing the samples obtained in step (b) or by placing the shaped bodies thereof between a positive electrode and a negative electrode, wherein the samples obtained in step (b) or the shaped bodies thereof are in contact with both electrodes. The electrodes may, by way of example, be in the form of stainless steel plates, in particular stainless steel AISI 304 plates. Further, the electrodes may have a mutual distance of 0.01 mm to 10 cm, in particular 0.01 mm to 5 cm, preferably 0.01 mm to 1 mm.

The electrodes can be of different shapes. The electrodes may have a polygonal cross-section, for example quadratic or rectangular, or a corner-less, in particular circular, oval-shaped or elliptical, cross-section. In particular, the shaped body may have a thickness of > 0 cm to 10 cm in particular > 0 cm to 5 cm, preferably > 0 cm to 1 mm. For example, the electrodes may be in the form of a disc, plate or a cylinder.

Further, the constant or variable DC voltage or the equivalent electric field may be applied in the aforementioned step (c) for 1 h to 24 h, in particular 0.1 h to 10 h, in particular 1 h.

Further, the DC voltage applied in the aforementioned step (c) is preferably 500 V, which is equivalent to a constant electric field of 3 kV/cm.

Further, the equivalent electric field applied in the aforementioned step (c) is preferably 3 kV/cm.

Further, the temperature in the aforementioned step (c) is preferably at least 900 °C, more preferably at least 1000 °C. Preferably, the temperature in step (c) is 900 °C to 1200 °C, in particular 1000 °C to 1200 °C, particularly 1000 °C.

Preferably, step (c) is carried out by applying a constant or variable DC voltage of 500 V at 1000 °C for 1 h to the samples obtained in step (b) or the shaped bodies, in particular discoidal shaped bodies, thereof.

Further, the aforementioned step (d) may be carried out by cooling the samples obtained in step (c) to room temperature.

Further, the aforementioned step (d) may be carried out for 1 min to 72 h, in particular 15 min to 5 h, preferably 15 min to 2 h.

In a further embodiment of the invention, the permanently polarized hydroxyapatite is obtained or obtainable by a process comprising the steps of
(a) preparing samples of hydroxyapatite, in particular crystalline hydroxyapatite, in particular using ammonium phosphate dibasic (diammonium hydrogen phosphate, (NH₄)₂HPO₄) and calcium nitrate (Ca(NO₃)₂) as reactants or starting materials,
(b) sintering the samples prepared in step (a), in particular at a temperature of 1000 °C, in particular for 2 h,
(c) applying an equivalent electric field of 3 kV/cm, in particular at a temperature of 1000 °C, in particular for 1 h, to the samples obtained in step (b) or to shaped bodies thereof and
(d) cooling the samples obtained in step (c) maintaining the equivalent electric field, in particular for 30 min.

With respect to further features and advantages of the steps (a)-(d), reference is made in its entirety to the previous description.

In a further embodiment of the invention, the contacting step is carried out in the presence of liquid water and/or water vapor. In other words, according to a further embodiment of the invention, the water is in liquid form and/or vapor form, for carrying out the contacting step.

In a further embodiment of the invention, the contacting step is carried out with a volumetric ratio of permanently polarized hydroxyapatite to water, in particular liquid water and/or water vapor, of 1000:1 to 0.01:1, in particular 500:1 to 100:1, preferably 300:1 to 350:1.

In a further embodiment of the invention, the contacting step is carried out with a volumetric ratio of carbon dioxide to methane of 200:1, in particular 3:1, preferably 1:1.

In a further embodiment of the invention, the contacting step is carried out under a total pressure of 0.1 bar to 100 bar, in particular 0.1 bar to 10 bar, in particular 1 bar to 10 bar, in particular 1 bar to 8 bar, in particular 1 bar to 6 bar, preferably of 6 bar.

The term "total pressure" as used according to the present invention refers to the sum of each gas partial pressure of the gas mixture, preferably at room temperature.

In a further embodiment of the invention, the contacting step is carried out under a pressure of carbon dioxide of 0.035 bar to 90 bar, in particular 0.1 bar to 10 bar, in particular 1 bar to 8 bar, preferably of 6 bar.

In a further embodiment of the invention, the contacting step is carried out under a partial pressure of carbon dioxide of 0.035 bar to 90 bar, in particular 0.1 bar to 3 bar, in particular 1 bar to 3 bar, preferably of 3 bar, and/or under a partial pressure of methane of 0.00017 bar to 5 bar, in particular 1 bar to 3 bar, preferably of 3 bar.

Further, the contacting step may be carried out with a total pressure of the gas mixture from 0.0001 bar to 250 bar in the presence of the catalyst and the water, in particular liquid water.

Further, the contacting step may be carried out with a pressure ratio of carbon dioxide to methane (CO₂ : CH₄) in the presence of the catalyst from 0.0001 bar : 250 bar to 250 bar : 0.0001 bar.

Further, the gas mixture may be in particular free of nitrogen (N₂). In other words, the contacting step may be carried out in the absence of nitrogen.

In a further embodiment of the invention, the contacting step is carried out with a molar ratio of carbon dioxide to permanently polarized hydroxyapatite of 0.1 to 0.5, in particular 0.2 to 0.5, preferably 0.3 to 0.5.

In a further embodiment of the invention, the contacting step is carried out with a molar ratio of methane to permanently polarized hydroxyapatite of 0.1 to 0.5, in particular 0.2 to 0.5, preferably 0.3 to 0.5.

Preferably, the contacting step is carried out by using an uncoated permanently polarized hydroxyapatite, i.e. by using a permanently polarized hydroxyapatite lacking any coating. In that regard, it surprisingly turned out that the application of an uncoated permanently polarized hydroxyapatite advantageously significantly increases the conversion of carbon dioxide into functionalized organic molecules having 2 and/or 3 carbon atoms (such as ethanol and/or acetic acid and/or acetone) and particularly in addition maximizes the selective synthesis of ethanol as the major reaction product. Similarly, the application of an uncoated permanently polarized hydroxyapatite advantageously significantly increases the conversion of carbon dioxide and methane into ethanol and particularly in addition maximizes the selective synthesis of ethanol as the major reaction product.

Alternatively, the contacting step may be carried out by using a coated permanently polarized hydroxyapatite. In principle, the contacting step may be carried out by using a permanently polarized hydroxyapatite being coated with an inorganic photocatalyst such as TiO₂, MgO₂, MnO₂ or combinations thereof. More specifically, the contacting step may be carried out by using a permanently polarized hydroxyapatite having a three-layered coating, in particular wherein the three-layered coating may be composed of two layers of aminotris(methylenephosphonic acid) and a layer of zirconium oxychloride (ZrOCl₂) or zirconia ZrO2, wherein the layer of zirconium oxychloride is arranged or sandwiched between the two layers of aminotris(methylenephosphonic acid). By using a coated permanently polarized hydroxyapatite, the efficiency of the reaction can be advantageously increased.

In a further embodiment of the invention, the contacting step is carried out under UV (ultraviolet) irradiation or UV-Vis (ultraviolet-visible) irradiation. In particular, the contacting step may be carried out under UV irradiation or UV-Vis irradiation having a wavelength from 200 nm to 850 nm, in particular 240 nm to 400 nm, preferably 250 nm to 260 nm, more preferably of 253.7 nm.

Further, the contacting step may be in particular carried out under UV irradiation having a wavelength from 200 nm to 280 nm, in particular 240 nm to 270 nm, preferably 250 nm to 260 nm, more preferably of 253.7 nm. Preferably, the permanently polarized hydroxyapatite is directly exposed to or irradiated with the UV irradiation or UV-Vis irradiation. Advantageously, the UV irradiation or UV-Vis irradiation are/is provided by a suitable UV source and/or Vis source, for example UV lamp and/or Vis lamp.

In a further embodiment of the invention, the contacting step is carried out under UV (ultraviolet) irradiation or UV-Vis (ultraviolet-visible) irradiation having a irradiance from 0.1 W/m² to 200 W/m², in particular 1 W/m² to 50 W/m², preferably 2 W/m² to 10 W/m², more preferably of 3 W/m². With respect to advantages of this embodiment, reference is made to the preceding paragraph.

In a further embodiment of the invention, the contacting step is carried out at a temperature of 25 °C to 250 °C, in particular 95 °C to 140 °C, preferably of 95 °C.

More preferably, the contacting step is carried out at a temperature of 95 °C and under UV irradiation. These reaction conditions are especially useful for synthesizing, in particular selectively synthesizing, functionalized organic molecules having 2 carbon atoms ( ethanol and/or acetic acid) in high yields.

Further, the contacting step may be preferably carried out without UV irradiation and at a temperature of 25 °C to 250 °C, in particular 95 °C to 140 °C, preferably of 140 °C. Also, the reaction conditions according to this embodiment result in the synthesis, in particular selective synthesis, of functionalized organic molecules having 2 carbon atoms (ethanol and/or acetic acid) in high yields.

Further, the contacting step may be carried out for 0.0001 h to 120 h, in particular 24 h to 72 h, preferably 48 h to 72 h.

Further, the process, in particular the contacting step, may be carried out continuously or discontinuously, in particular as a batch process.

Further, the contacting step may be carried out by using air, in particular traffic contaminated air, as gas mixture. Thus, it is possible to synthesize functionalized organic molecules having 1 to 3 carbon atoms, in particular ethanol and/or acetic acid and/or methanol and/or formic acid and/or acetone, as valuable compounds and concurrently to remove carbon dioxide from air, in particular traffic contaminated air.

Preferably, the process comprises a further step
- isolating and/or separating and/or purifying the functionalized organic molecules obtained during or in the contacting step.

The above further step is preferably carried out by dissolving and extracting the catalyst and/or by extracting a supernatant formed during or in the contacting step.

In a further embodiment of the invention, the process is used for producing or synthesizing, in particular selectively producing or synthesizing, ethanol.

In a further embodiment of the invention, the process is used for producing or synthesizing a mixture comprising or consisting of ethanol and at last one further functionalized organic molecule, preferably selected from the group consisting of methanol, formic acid, acetic acid, malonic acid and acetone.

More preferably, the process is used for producing or synthesizing a mixture comprising or consisting of ethanol and at last one further functionalized organic molecule selected from the group consisting of methanol, formic acid, acetic acid and acetone.

Alternatively, the process is preferably used for producing or synthesizing a mixture comprising or consisting of ethanol and at last one further functionalized organic molecule selected from the group consisting of methanol, acetic acid, malonic acid and acetone.

In a further embodiment of the invention, the process is used for producing or synthesizing a mixture comprising or consisting of ethanol, methanol, formic acid, acetic acid and acetone.

In a further embodiment of the invention, the process is used for producing or synthesizing a mixture comprising or consisting of ethanol, methanol, acetic acid, malonic acid and acetone.

Further, the present invention relates to the use of the process according to the present invention for removing carbon dioxide from an atmosphere, in particular from air, i.e. atmosphere of Earth. In particular, the present invention relates to the use of the process according to the present invention for removing carbon dioxide from polluted or contaminated air such as traffic contaminated air.

The term "air" or "atmosphere of Earth" as used according to the present invention means a layer of gases retained by Earth's gravity, surrounding the planet's Earth and forming its planetary atmosphere.

With respect to further features and advantages of the use, reference is made in its entirety to the previous description.

Finally, the present invention relates to the use of a process comprising the step of
- contacting a gas mixture comprising or consisting of carbon dioxide (CO₂) and methane (CH₄), in particular only comprising or consisting of carbon dioxide (CO₂) and methane (CH₄), in presence of water, in particular liquid water, (H₂O) with a catalyst, in particular electrocatalyst, comprising or consisting of permanently polarized hydroxyapatite
for the production or synthesis, in particular selective production or synthesis, of organic molecules having 1 to 3 carbon atoms, wherein the functionalized organic molecules are selected from the group consisting of ethanol, methanol, formic acid, acetic acid, malonic acid, acetone and a mixture of at least two of the afore-said functionalized organic molecules.

Preferably, said use of the process is for the production or synthesis, in particular selective production or synthesis, of ethanol or a mixture comprising or consisting of ethanol and at least one further functionalized organic molecule selected from the group consisting of methanol, formic acid, acetic acid, malonic acid and acetone, in particular to a mixture comprising or consisting of ethanol, methanol, formic acid, acetic acid and acetone, in particular with ethanol as a major reaction product, or to a mixture comprising or consisting of ethanol, methanol, acetic acid, malonic acid and acetone, in particular with ethanol as a major reaction product. With respect to further features and advantages of the use, in particular in terms of the process and the functionalized organic molecules, reference is made in its entirety to the previous description.

Further features and advantages of the invention will become clear from the following examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

### DESCRIPTION OF THE FIGURES

For a better understanding of what has been disclosed, some figures are attached which schematically or graphically and solely by way of non-limiting example show a practical case of embodiment of the present invention.
Fig. 1 graphically shows a ³¹P-NMR spectrum of the permanently polarized hydroxyapatite (p-HAp) according to the present invention.
Fig. 2(a) schematically depicts the Raman spectra of hydroxyapatite (HAp) samples with the deconvolution of the v₁ peak in the 930-990 cm⁻¹ interval. Counts (A.U.) are plotted on the ordinate. Raman shift (cm⁻¹) is plotted on the abscissa.
Fig. 2(b) schematically depicts the Raman spectra of permanently polarized hydroxyapatite (p-HAp) samples with the deconvolution of the v₁ peak in the 930-990 cm⁻¹ interval. Counts (A.U.) are plotted on the ordinate. Raman shift (cm⁻¹) is plotted on the abscissa.

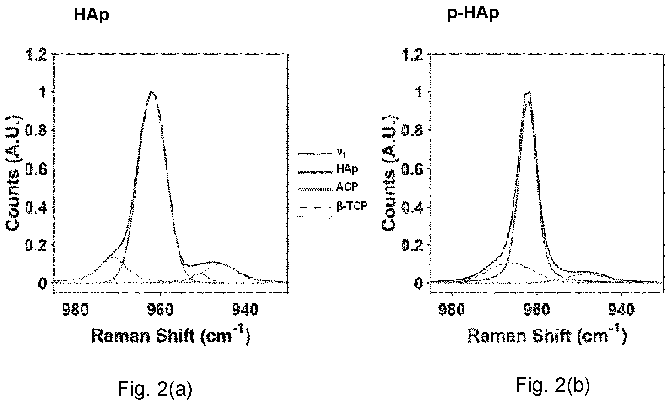

The Figs. 2(a)-(b), which compare the Raman v₁ peak in the 930-990 cm⁻¹ interval before and after polarization of Hap, prove the success of the polarization process.
   The area of HAp, amorphous calcium phosphate (ACP) and β-tricalcium phosphate (β-TCP) indicates the content of each phase. The content of co-existing phases experiences a reduction in polarized samples (i.e. 4.3 % and 9.8 % for ACP and β-TCP, respectively) that is accompanied by a decrease of the full width at half maximum (FWHM) from 9 cm⁻¹ in HAp to 5 cm⁻¹ in p-HAp. This result indicates an increase of the HAp phase by means of a reduction of crystal imperfections, such as PO₄³⁻ tetrahedrons distortions.

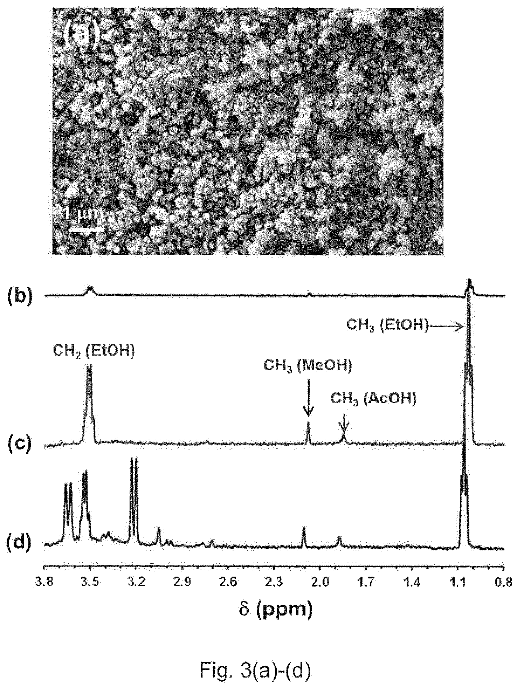

Fig. 3(a) shows a scanning electron microscopy micrograph of the permanently polarized hydroxyapatite. Accordingly, the permanently polarized hydroxyapatite can be described as particles of (approximately) 100 nm to 300 nm that aggregate forming agglomerates of up to 1 µm in size.
Fig. 3(b) graphically shows the ¹H-NMR spectrum of a solution obtained after extraction of the reaction products of a reaction that was conducted for 72 h using CO₂ (3 bar), CH₄ (3 bar), H₂O (1 mL) in the presence of conventional (i.e. non-polarized) hydroxyapatite as a catalyst, at 95 °C under UV radiation. The reacted catalyst was dissolved in an aqueous solution with 100 mM HCI and 50 mM NaCl.
Fig. 3(c) graphically shows the ¹H-NMR spectrum of a solution obtained after extraction of the reaction products of a reaction that was conducted for 72 h using CO₂ (3 bar), CH₄ (3 bar), H₂O (1 mL) using (uncoated) permanently polarized hydroxyapatite as catalyst, at 95 °C under UV radiation. The reacted catalyst was dissolved in an aqueous solution with 100 mM HCI and 50 mM NaCl.
Fig. 3(d) graphically shows the ¹H-NMR spectrum of a solution obtained after extraction of the reaction products of a reaction that was conducted for 72 h using CO₂ (3 bar), CH₄ (3 bar), H₂O (1 mL) in the presence of coated p-HAp. The p-HAp was coated with aminotris(methylenephosphonic acid), hereafter denoted as ATMP, and zirconium oxychloride (ZrOCl₂), hereafter denoted as ZC, at 95 °C under UV radiation. The reacted catalyst was dissolved in an aqueous solution with 100 mM HCI and 50 mM NaCl.
   As is shown in figs. 3(b)-(d), chemical shifts observed after the dissolution of the coated p-HAp are slightly deshielded with respect to the peaks of products derived from non-coated catalysts. This effect has been attributed to the aminotris(methylenephosphonic acid) (ATMP), which increases the acidity of the medium, causing downfield shifts that are not detected for p-HAp and HAp, independently of the conditions. On the other hand, figs. 3(b)-(c) indicate that the elimination of the coating from the catalyst not only increases the conversion into ethanol by 20 %, but also maximizes the selective synthesis of ethanol as the major reaction product.

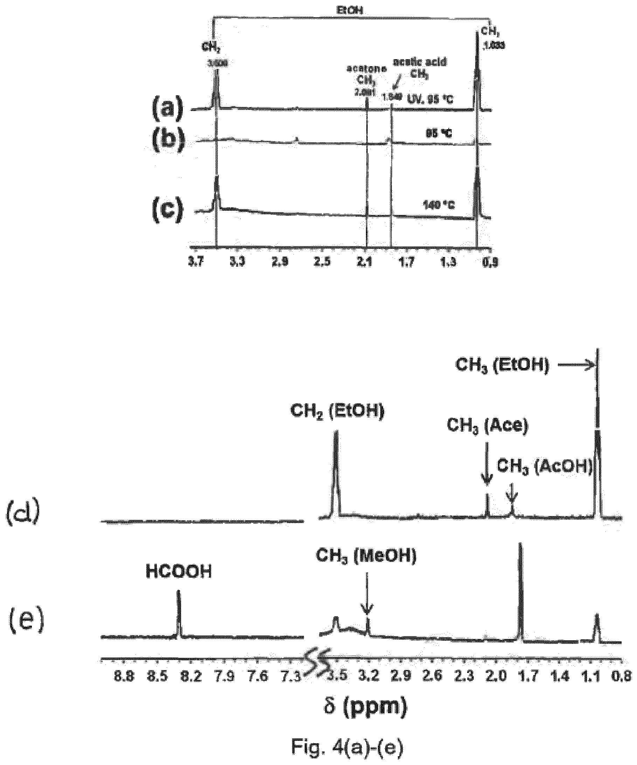

Fig. 4(a) graphically shows a further ¹H-NMR spectrum of a solution obtained after extraction of the reaction products achieved after 72 h from CO₂ (3 bar), CH₄ (3 bar) and H₂O (1 mL) using (uncoated) permanently polarized hydroxyapatite as catalyst and 95 °C and UV radiation as reaction conditions. The catalyst was dissolved in an aqueous solution with 100 mM HCI and 50 mM NaCl.
Fig. 4(b) graphically shows the ¹H-NMR spectrum of a solution obtained after extraction of the reaction products achieved after 72 h from CO₂ (3 bar), CH₄ (3 bar) and H₂O (1 mL) using (uncoated) permanently polarized hydroxyapatite as catalyst and 95 °C without UV radiation as reaction conditions. The catalyst was dissolved in an aqueous solution with 100 mM HCI and 50 mM NaCl.
Fig. 4(c) graphically shows the ¹H-NMR spectrum of a solution obtained after extraction of the reaction products achieved after 72 h from CO₂ (3 bar), CH₄ (3 bar) and H₂O (1 mL) using permanently polarized hydroxyapatite as catalyst and 140 °C without UV radiation as reaction conditions. The catalyst was dissolved in an aqueous solution with 100 mM HCI and 50 mM NaCl.
Fig. 4(d) graphically shows a further ¹H-NMR spectrum of the liquid water after reaction for 72 h from CO₂ (3 bar), CH₄ (3 bar) and H₂O (1 mL) using (uncoated) permanently polarized hydroxyapatite as catalyst and 95 °C and UV radiation as reaction conditions. Spectra were cut to avoid the very intense peak of water at 4.7 ppm.
Fig. 4(e) graphically shows a further ¹H-NMR spectrum of a solution obtained after extraction of the reaction products achieved after 72 h from CO₂ (3 bar), CH₄ (3 bar) and H₂O (1 mL) using (uncoated) permanently polarized hydroxyapatite as catalyst and 95 °C and UV radiation as reaction conditions. The catalyst was dissolved in an aqueous solution with 100 mM HCI and 50 mM NaCl. Spectra were cut to avoid the very intense peak of water at 4.7 ppm.
   The spectra reveal the apparition of methanol and formic acid as reaction products in the liquid water used for the reaction. Ethanol and acetic acid appears in both the catalyst and the liquid water, while acetone is only detected in the former.

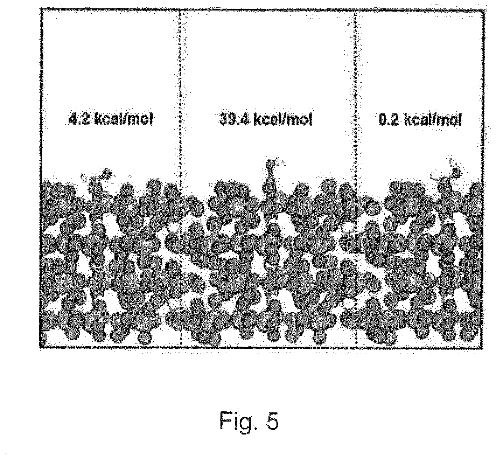

Fig. 5 graphically shows representation of three protonated forms of CO₂ adsorbed molecules on the OH⁻ vacancy of permanently polarized hydroxyapatite. The numeric values (in eV) stand for the calculated adsorption energies.
   In order to support the p-HAp fixation mechanism based on the formation of carboxylates, DFT calculations were performed at the PBE-D3 level. Calculations were performed considering the (0001) facet, which is the most stable HAp surface, and considering an isodesmic model in which H₂ is used as a source of protons. The adsorption energies of three different protonation products of CO₂ were calculated by inserting the molecules in the hydroxyl vacancy of the mineral. Results proved that the protonation of CO₂ to formic acid is exothermic in the gas phase by -3.1 kcal/mol, but it is more exothermic when adsorbed on p-HAp substrate, by -32.7 kcal/mol. Yet, all protonated species display endothermic adsorption energies, the one for the protonated formic acid is very small (0.2 kcal/mol) while the one for the CO₂ is 5.1 kcal/mol (other sites were checked on the p-HAp displaying higher energies, as shown for some representative cases in fig. 5), thus making this pathway unfeasible to be followed completely and shifting the catalysis location elsewhere close to.

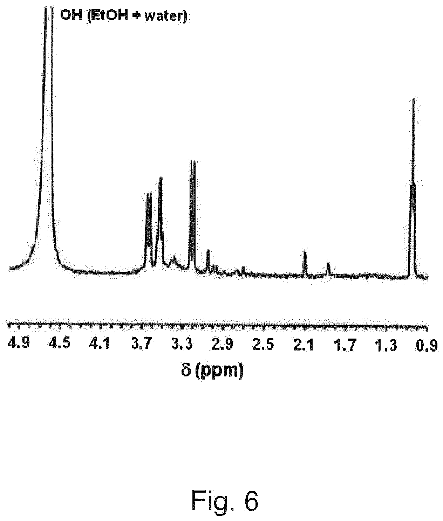

Fig. 6 graphically shows a further ¹H-NMR spectrum of a solution obtained after extraction of the reaction products achieved after 72 h from CO₂ (3 bar), CH₄ (3 bar) and H₂O (1 mL) in the presence of permanently polarized hydroxyapatite coated with aminotris(methylenephosphonic acid) and zirconium oxychloride, at 95 °C under UV radiation. The reacted catalyst was dissolved in an aqueous solution with 100 mM HCI and 50 mM NaCl. The spectrum includes the OH band at 4.65 ppm.

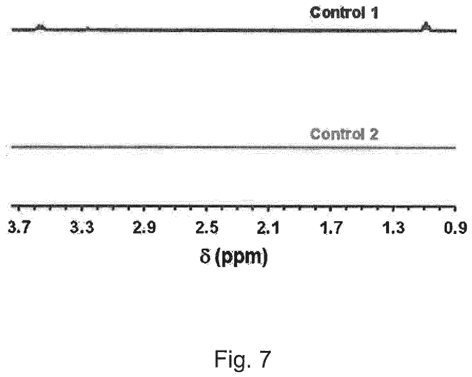

Fig. 7 graphically shows the ¹H-NMR spectrum of the liquid water after 72 h from CO₂ (3 bar), CH₄ (3 bar) and H₂O (1 mL) at 95 °C using UV radiation (control 1) and without using UV radiation (control 2). No catalyst was used for this reaction.

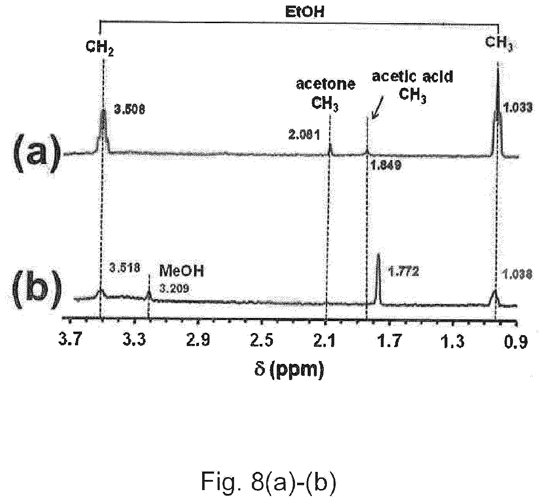

Fig. 8(a) shows a further ¹H-NMR spectrum of the reaction products achieved after 72 h from CO₂ (3 bar), CH₄ (3 bar) and H₂O (1 mL) at 95 °C using UV radiation and (uncoated) permanently polarized hydroxyapatite as catalyst, wherein the analysis of the solution obtained after extraction of the products was performed by dissolving the catalyst with 100 mM HCI and 50 mM NaCl.
Fig. 8(b) graphically shows the ¹H-NMR spectra of the reaction products achieved after 72 h from CO₂ (3 bar), CH₄ (3 bar) and H₂O (1 mL) at 95 °C using UV radiation and using (uncoated) permanently polarized hydroxyapatite as catalyst, wherein liquid water, which has been incorporated to the reaction chamber, has been analyzed.

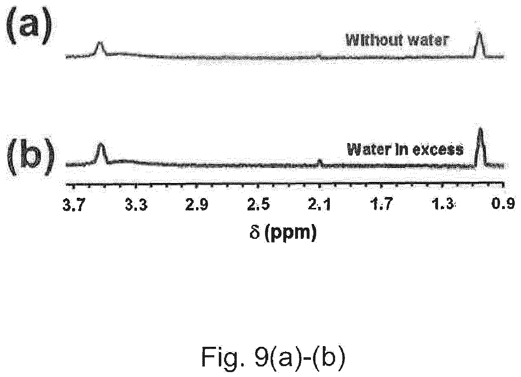

Fig. 9(a) graphically shows the ¹H-NMR spectrum of the reaction products achieved after 72 h from CO₂ (3 bar) and CH₄ (3 bar) at 95 °C using UV radiation and (uncoated) permanently polarized hydroxyapatite as catalyst in absence of water.
Fig. 9(b) graphically shows the ¹H-NMR spectrum of the reaction products achieved after 72 h from CO₂ (3 bar) and CH₄ (3 bar) at 95 °C using UV radiation and (uncoated) permanently polarized hydroxyapatite as catalyst with an excess of water.

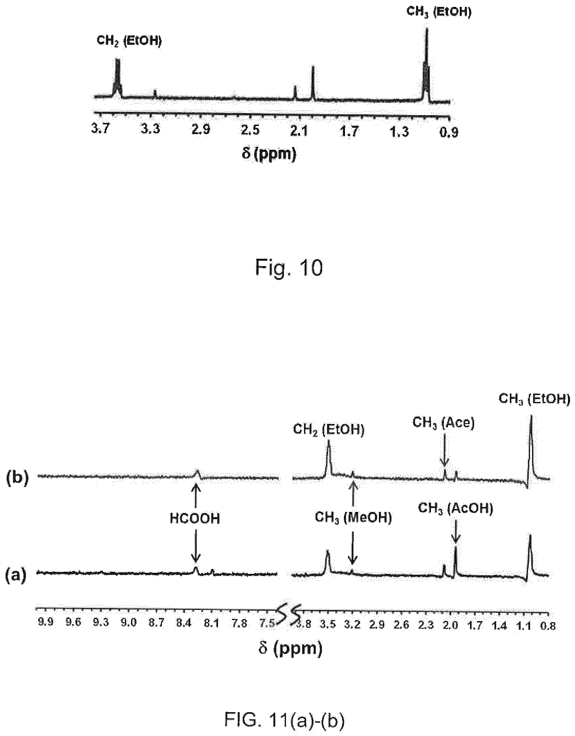

Fig. 10 graphically shows the ¹H-NMR spectrum of the solution obtained after extraction of the reaction products achieved after 72 h from polluted air (atmospheric pressure) and H₂O (1 mL) at 95 °C using (uncoated) permanently polarized hydroxyapatite as catalyst at 95 °C and under UV radiation. The reacted catalyst was dissolved in an aqueous solution with 100 mM HCI and 50 mM NaCl.
Fig. 11(a) graphically shows a further ¹H-NMR spectrum of the liquid water after reaction for 48 h from CO₂ (6 bar) and H₂O (1 mL) using (uncoated) permanently polarized hydroxyapatite as catalyst and 140 °C as reaction conditions (without UV radiation). Spectra were cut to avoid the very intense peak of water at 4.7 ppm.
Fig. 11(b) graphically shows a further ¹H-NMR spectrum of a solution obtained after extraction of the reaction products achieved after 48 h from CO₂ (6 bar) and H₂O (1 mL) using (uncoated) permanently polarized hydroxyapatite as catalyst and 140 °C as reaction conditions (without UV radiation). The catalyst was dissolved in an aqueous solution with 100 mM HCI and 50 mM NaCl. Spectra were cut to avoid the very intense peak of water at 4.7 ppm.
   The spectra reveal the apparition of methanol, formic acid, ethanol, acetic acid and acetone as reaction products in both the liquid water and the catalyst and the liquid water. The yields (µmol/g of catalyst) in the liquid water were: 0.21 ± 0.07 (methanol), 2.44 ± 0.97 (formic acid), 4.50 ± 0.91 (ethanol), 2.22 ± 0.88 (acetic acid) and 0.74 ± 0.15 (acetone). The yields (µmol/g of catalyst) in the catalyst were: 0.56 ± 0.19 (methanol), 3.22 ± 0.54 (formic acid), 6.60 ± 2.32 (ethanol), 0.49 ± 0.12 (acetic acid) and 0.62 ± 0.27 (acetone).

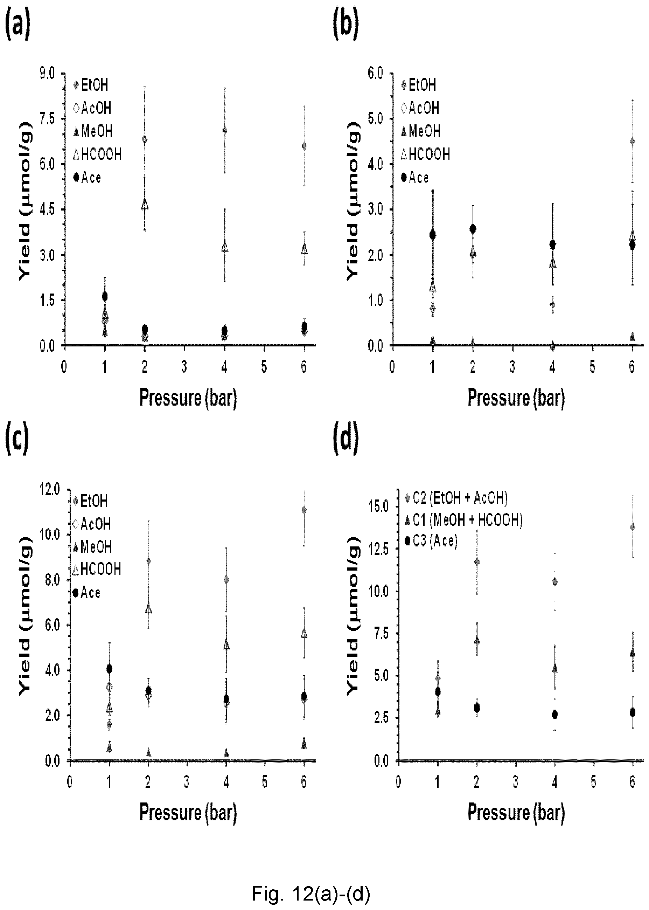

Fig. 12(a) graphically shows the variation of the yield (expressed as µmol of product / g of catalyst) of ethanol (EtOH), acetic acid (AcOH), methanol (MeOH), formic acid (HCOOH) and acetone (Ace) against the CO₂ pressure (in bars) as determined by ¹H NMR spectroscopy from the solution obtained after extraction of the reaction products achieved after 48 h using CO₂ (1, 2, 4 or 6 bars) and H₂O (1 mL) at 140 °C (without UV radiation). The catalyst was dissolved in an aqueous solution with 100 mM HCI and 50 mM NaCl.
Fig. 12(b) graphically shows the variation of the yield (expressed as µmol of product / g of catalyst) of ethanol (EtOH), acetic acid (AcOH), methanol (MeOH), formic acid (HCOOH) and acetone (Ace) against the CO₂ pressure (in bars) as determined by ¹H NMR spectroscopy from the liquid water using (uncoated) permanently polarized hydroxyapatite as catalyst. In all cases the reaction was conducted for 48 h using CO₂ (1, 2, 4 or 6 bars) and H₂O (1 mL) at 140 °C (without UV radiation).
Fig. 12(c) graphically shows the variation of the sum of the yields (expressed as µmol of product / g of catalyst) achieved from the solution obtained after extraction of the reaction products from the catalyst (Fig. 12a) and the supernatant (Fig. 12b) for ethanol (EtOH), acetic acid (AcOH), methanol (MeOH), formic acid (HCOOH) and acetone (Ace) against the CO₂ pressure (in bars). In all cases the reaction was conducted for 48 h using CO₂ (1, 2, 4 or 6 bars) and H₂O (1 mL) at 140 °C (without UV radiation).
Fig. 12(d) graphically shows the variation of the sum of the yields (expressed as µmol of product / g of catalyst) achieved from the solution obtained after extraction of the reaction products from the catalyst (Fig. 12a) and supernatant (Fig. 12b) for C1 (methanol and formic acid; MeOH+HCOOH), C2 (ethanol and acetic acid; EtOH+AcOH) and C3 (acetone; Ace) against the CO₂ pressure (in bars). In all cases the reaction was conducted for 48 h using CO₂ (1, 2, 4 or 6 bars) and H₂O (1 mL) at 140 °C (without UV radiation).

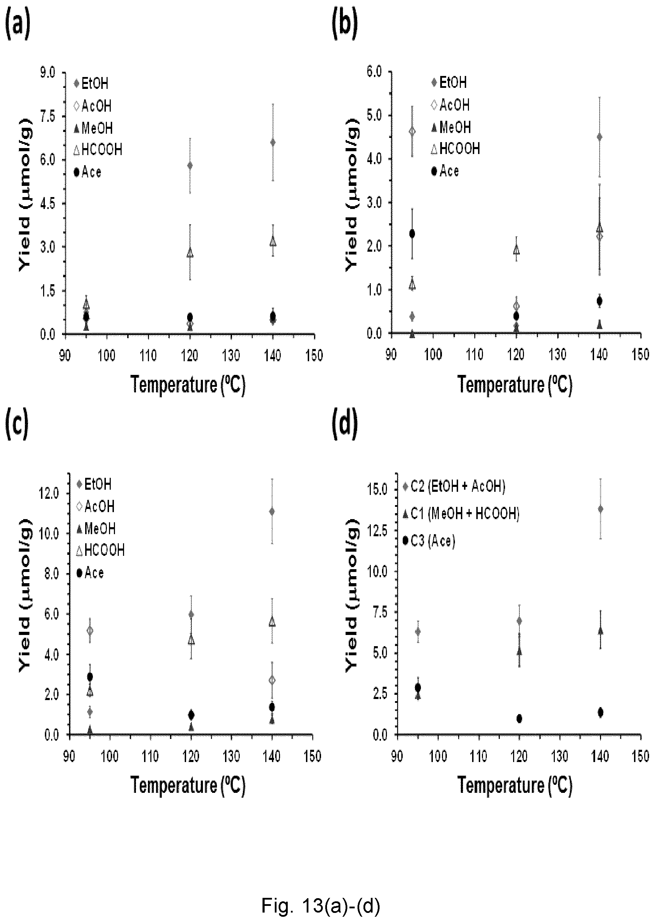

Fig. 13(a) graphically shows the variation of the yield (expressed as µmol of product / g of catalyst) of ethanol (EtOH), acetic acid (AcOH), methanol (MeOH), formic acid (HCOOH) and acetone (Ace) against the temperature (in °C) as determined by ¹H NMR spectroscopy from the solution obtained after extraction of the reaction products achieved after 48 h using CO₂ (6 bars) and H₂O (1 mL) at 95, 120 or 140 °C (without UV radiation). The catalyst was dissolved in an aqueous solution with 100 mM HCI and 50 mM NaCl.
Fig. 13(b) graphically shows the variation of the yield (expressed as µmol of product / g of catalyst) of ethanol (EtOH), acetic acid (AcOH), methanol (MeOH), formic acid (HCOOH) and acetone (Ace) against the temperature (in °C) as determined by ¹H NMR spectroscopy from the liquid water using (uncoated) permanently polarized hydroxyapatite as catalyst. In all cases the reaction was conducted for 48 h using CO₂ (6 bars) and H₂O (1 mL) at 95, 120 or 140 °C (without UV radiation).
Fig. 13(c) graphically shows the variation of the sum of the yields (expressed as µmol of product / g of catalyst) achieved from the solution obtained after extraction of the reaction products from the catalyst (Fig. 13a) and the supernatant (Fig. 13b) for ethanol (EtOH), acetic acid (AcOH), methanol (MeOH), formic acid (HCOOH) and acetone (Ace) against the temperature (in °C). In all cases the reaction was conducted for 48 h using CO₂ (6 bars) and H₂O (1 mL) at 95, 120 or 140 °C (without UV radiation).
Fig. 13(d) graphically shows the variation of the sum of the yields (expressed as µmol of product / g of catalyst) achieved from the solution obtained after extraction of the reaction products from the catalyst (Fig. 13a) and supernatant (Fig. 13b) for C1 (methanol and formic acid; MeOH+HCOOH), C2 (ethanol and acetic acid; EtOH+AcOH) and C3 (acetone; Ace) against the temperature (in °C). In all cases the reaction was conducted for 48 h using CO₂ (6 bars) and H₂O (1 mL) at 95, 120 or 140 °C (without UV radiation).

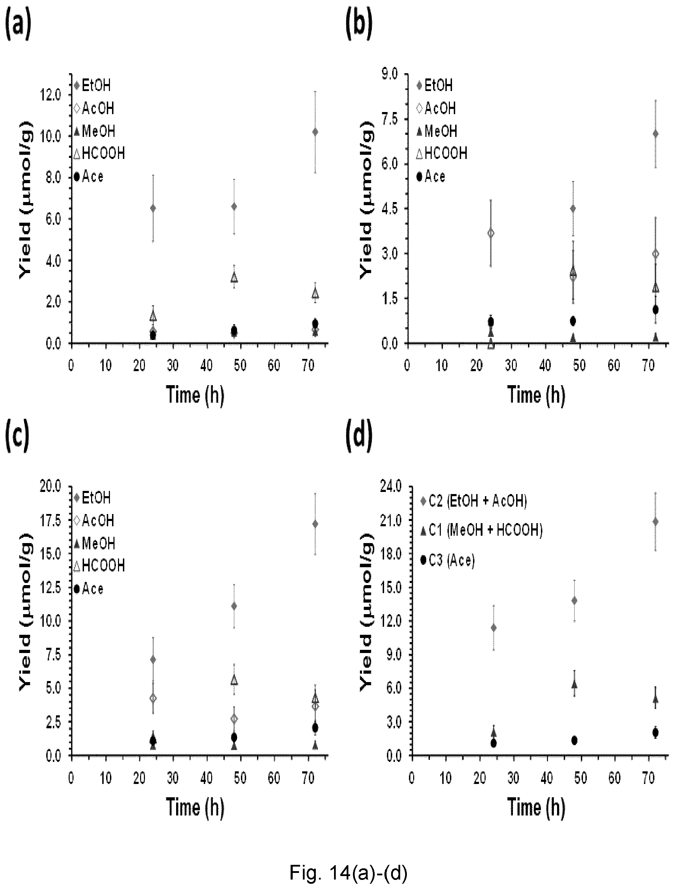

Fig. 14(a) graphically shows the variation of the yield (expressed as µmol of product / g of catalyst) of ethanol (EtOH), acetic acid (AcOH), methanol (MeOH), formic acid (HCOOH) and acetone (Ace) against the reaction time (in hours) as determined by ¹H NMR spectroscopy from the solution obtained after extraction of the reaction products achieved after 24, 48 and 72 h using CO₂ (6 bars) and H₂O (1 mL) at 140 °C (without UV radiation). The catalyst was dissolved in an aqueous solution with 100 mM HCI and 50 mM NaCl.
Fig. 14(b) graphically shows the variation of the yield (expressed as µmol of product / g of catalyst) of ethanol (EtOH), acetic acid (AcOH), methanol (MeOH), formic acid (HCOOH) and acetone (Ace) against the time (in hours) as determined by ¹H NMR spectroscopy from the liquid water using (uncoated) permanently polarized hydroxyapatite as catalyst. In all cases the reaction was conducted for 24, 48 or 72 h using CO₂ (6 bars) and H₂O (1 mL) at 140 °C (without UV radiation).
Fig. 14(c) graphically shows the variation of the sum of the yields (expressed as µmol of product / g of catalyst) achieved from the solution obtained after extraction of the reaction products from the catalyst (Fig. 14a) and the supernatant (Fig. 14b) for ethanol (EtOH), acetic acid (AcOH), methanol (MeOH), formic acid (HCOOH) and acetone (Ace) against the time (in hours). In all cases the reaction was conducted for 24, 48 or 72 h using CO₂ (6 bars) and H₂O (1 mL) at 140 °C (without UV radiation).
Fig. 14(d) graphically shows the variation of the sum of the yields (expressed as µmol of product / g of catalyst) achieved from the solution obtained after extraction of the reaction products from the catalyst (Fig. 14a) and supernatant (Fig. 14b) for C1 (methanol and formic acid; MeOH+HCOOH), C2 (ethanol and acetic acid; EtOH+AcOH) and C3 (acetone; Ace) against the time (in hours). In all cases the reaction was conducted for 24, 48 or 72 h using CO₂ (6 bars) and H₂O (1 mL) at 140 °C (without UV radiation).

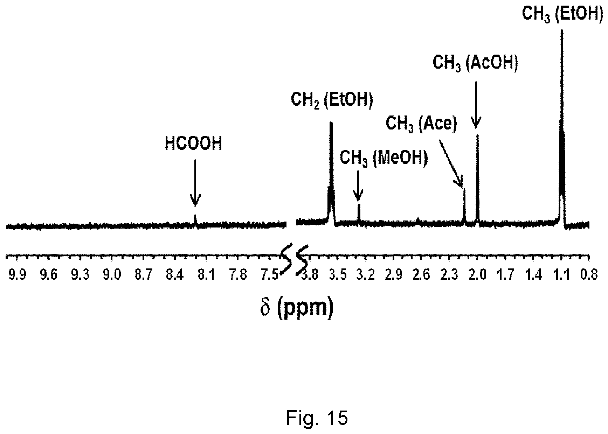

Fig. 15 graphically shows the ¹H-NMR spectrum of the solution obtained after extraction of the reaction products achieved after 72 h from polluted air (atmospheric pressure) and H₂O (1 mL) at 95 °C using (uncoated) permanently polarized hydroxyapatite as catalyst at 95 °C and under UV radiation. The reacted catalyst was dissolved in an aqueous solution with 100 mM HCI and 50 mM NaCl.

### EXPERIMENTAL SECTION

### 1. Materials

Calcium nitrate (Ca(NO₃)₂), diammonium hydrogen phosphate ((NH4)₂HPO₄; purity > 99.0 %) and ammonium hydroxide solution 30 % (NH₄OH; purity: 28-30% w/w) were purchased from Sigma Aldrich. Ethanol (purity > 99.5 %) was purchased from Scharlab. All experiments were performed with milli-Q water.

### 2. Hydrothermal synthesis of hydroxyapatite (HAp)

15 mL of 0.5 M of NH4)₂HPO₄ in de-ionized water were added at a rate of 2 mL min⁻¹ to 25 mL of 0.5 M of Ca(NO₃)₂ in ethanol (with pH previously adjusted to 10.5 using ammonium hydroxide solution) and left aging for 1 h. The whole process was performed under gentle agitation (150 rpm) and at room temperature. Hydrothermal treatment at 150 °C was applied using an autoclave Digestec DAB-2 for 24 h. The autoclave was allowed to cool down before opening. The precipitates were separated by centrifugation and washed with water and a 60/40 v/v mixture of ethanol-water (twice). After freeze-drying it for three days, the white powder obtained was sintered for 2 h at 1000 °C in air using the Carbolite ELF11/6W/301 furnace.

### 3. Thermally stimulated polarization process (TSP)

Mechanical consistent discs of around 1.5 mm of thickness were obtained by pressing 150 mg of previously sintered HAp powder at 620 MPa for 10 min. Thermal polarization was done placing the HAp discs between two stainless steel (AISI 304) and applying 3 kV/cm at 1000 °C for 1 h with a GAMMA power supply at 1000 °C using the same laboratory furnace as mentioned above. The discs were allowed to cool down maintaining the applied electric potential for 30 minutes, and finally, all the system was powered off and left to cool overnight.

### 4. Characterization

Vibrational spectra for a structural fingerprint were obtained by the inVia Qontor confocal Raman microscope (Renishaw), equipped with a Renishaw Centrus 2957T2 detector and a 785 nm laser.

SEM images were obtained using a Zeiss Neon 40 microscope equipped with a SEM GEMINI column. HRTEM was performed in a JEOL 2010F microscope equipped with a field emission electron source and operated at an accelerating voltage of 200 kV. The point-to-point resolution was 0.19 nm, and the resolution between lines was 0.14 nm. Samples were dispersed in an alcohol suspension in an ultrasonic bath, and a drop of the suspension was placed over a grid with holey-carbon film. Images were not filtered or treated by means of digital processing and they correspond to raw data. All ¹H-NMR spectra were acquired with a Bruker Avance III-400 spectrometer operating at 400.1 MHz and accumulating sixty-four scans. The chemical shift calibration was carried out using tetramethylsilane as internal standard. The samples were dissolved in milli-Q water containing 100 mM of HCI and 50 mM NaCl with the final addition of deuterated water.

### 5. Computational details

The 2 × 1 × 2 HAp supercell was chosen to build the (0001) facet for p-HAp. The latter was built by removing an OH⁻ orthonormal to the surface from the HAp supercell, which was previously optimized at the chosen DFT level. Consequently, a +1 global charge was applied for all calculations except for those involving formate, unpaired spin being considered when necessary. The initial coordinates of HAp were optimized following the computational details provided below to unwind surface tensions. The plane waves approach implemented in the Quantum Espresso 4.6 suite of Open-Source computer codes was used. Calculations were performed at the PBE level of theory corrected with the Grimme three body dispersion potentials (PBE-D3), applying the default C₆ software coefficients. A kinetic energy cutoff for the wave functions of 40 Ry was employed. A k-point mesh of 3 × 3 × 1 was automatically generated. Instead, a Gamma-center 1 × 1 × 1 k-mesh was used for calculations of discrete molecules and a 7 × 7 × 7 k-mesh for the bulk HAp calculations. Geometry optimizations were performed using the conjugated gradient algorithm until both the energy and force variation between consecutive steps was below 10⁻³ a.u and 10⁻⁴ a.u, respectively. The energy at each step was optimized until the deviation from self-consistency was below 10⁻⁵ Ry. Adsorption energies were calculated according to the following process: A+S → AS*, where A is the adsorbate; S the surface and AS* the adsorbed state. The adsorption energy (E_{ads}) was expressed as E_{ads}=E_{AS*} - (E_{A} + Eₛ).

### 6. Reaction chamber

A high pressure stainless steel reactor, which was designed ad hoc, was used to perform all the reactions. In brief, the reactor was dotted with a manometer, an electric heater with a thermocouple and an external temperature controller. The reactor was also characterized by an inert reaction chamber coated with a perfluorinated polymer (Teflon, 120 mL), where both the catalyst and water were incorporated. The reactor was equipped with three independent inlet valves for the incorporation of gases and an outlet valve to recover the gaseous reaction products. A UV lamp (GPH265T5L/4, 253.7 nm) was also placed in the middle of the reactor to irradiate the catalyst directly, the lamp being protected by a UV transparent quartz tube. All surfaces were coated with a thin film of perfluorinated polymer (Teflon) in order to avoid any contact between the reaction medium and the reactor surfaces, in this way discarding other catalyst effects.

### 7. Synthesis of coated p-HAp

Three-layered systems consisting of the successive deposition of aminotris(methylenephosphonic acid) (ATMP) and zirconium oxychloride (ZC) onto p-HAp were obtained by immersion in the corresponding aqueous solutions at room temperature for 5 h. In order to deposit a first ATMP layer, p-HAp was immersed into a 5 mM ATMP solution for 5 h. Subsequently, ZC was deposited onto the ATMP layered p-HAp by immersing the latter into a 5 mM ZrOCl₂ solution for 5 h. Finally, a second layer of ATMP was deposited on the ZC and ATMP layered p-HAp by immersing the latter into a 1.25 mM ATMP solution for 5 h.

### 8. Synthesis of functionalized organic molecules having 1 to 3 carbon atoms using uncoated p-HAp as catalyst

Functionalized organic molecules having 1 to 3 carbon atoms were synthesized from CO₂ gas alone (1, 2, 4 or 6 bars) as well as from CO₂ and CH₄ gas mixture (3 bar each) in the presence of uncoated p-HAp as catalyst and in the presence of liquid H₂O (1 mL). The reaction was carried out for 24, 48 or 72 h at 95, 120 or 140 °C and under irradiation of an UV lamp (GPH265T5L/4, 253.7 nm) illuminating directly the uncoated p-HAp or without UV radiation.

As representative examples of reactions, the following yields (expressed as µmol of product per gram of catalyst) were obtained:
- Reaction conducted for 72 h using CO₂ (3 bar), CH₄ (3 bar) and H₂O (1 mL) at 95 °C under UV radiation.

Yields obtained from the solution obtained after extraction by dissolving the catalyst: ethanol (19.4 ± 3.8 µmol/g), acetone (0.9 ± 0.1 µmol/g) and acetic acid (0.6 ± 0.1 µmol/g). Methanol and formic acid were not detected.

Yields obtained from the liquid water (supernatant): ethanol (0.7 ± 0.14 µmol/g), acetic acid (2.0 ± 0.5 µmol/g), methanol (1.5 ± 0.3 µmol/g) and formic acid (1.9 ± 0.6 µmol/g). Acetone was not detected.
- Reaction conducted for 48 h using CO₂ (6 bar) and H₂O (1 mL) at 140 °C without UV radiation.

Yields obtained from the solution obtained after extraction by dissolving the catalyst: ethanol (6.6 ± 2.3 µmol/g), formic acid (3.2 ± 0.5 µmol/g), acetone (0.6 ± 0.3 µmol/g), methanol (0.6 ± 0.2 µmol/g) and acetic acid (0.5 ± 0.1 µmol/g).

Yields obtained from the liquid water (supernatant): ethanol (4.5 ± 0.9 µmol/g), formic acid (2.4 ± 1.0 µmol/g), acetic acid (2.2 ± 0.9 µmol/g), acetone (0.7 ± 0.1 µmol/g) and methanol (0.2 ± 0.1 µmol/g).
- Reaction conducted for 48 h using CO₂ (1 bar) and H₂O (1 mL) at 140 °C without UV radiation.

Yields obtained from the solution obtained after extraction by dissolving the catalyst: acetone (1.6 ± 0.6 µmol/g), formic acid (1.1 ± 0.3 µmol/g), ethanol (0.8 ± 0.2 µmol/g), acetic acid (0.8 ± 0.2 µmol/g) and methanol (0.5 ± 0.2 µmol/g).

Yields obtained from the liquid water (supernatant): acid acetic (2.4 ± 1.0 µmol/g), formic acid (1.3 ± 0.3 µmol/g), acid formic (1.1 ± 0.3 µmol/g), acetone (0.8 ± 0.3 µmol/g), ethanol (0.8 ± 0.1 µmol/g) and methanol (0.1 ± 0.03 µmol/g).
- Reaction conducted for 48 h using CO₂ (6 bar) and H₂O (1 mL) at 95 °C without UV radiation.

Yields obtained from the solution obtained after extraction by dissolving the catalyst: formic acid (1.1 ± 0.3 µmol/g), ethanol (0.7 ± 0.3 µmol/g), acetone (0.6 ± 0.2 µmol/g), acetic acid (0.5 ± 0.1 µmol/g) and methanol (0.3 ± 0.1 µmol/g).

Yields obtained from the liquid water (supernatant): acid acetic (4.6 ± 0.6 µmol/g), acetone (2.3 ± 0.3 µmol/g), formic acid (1.1 ± 0.1 µmol/g), and ethanol (0.4 ± 0.1 µmol/g). Methanol was not detected.
- Reaction conducted for 72 h using CO₂ (6 bar) and H₂O (1 mL) at 140 °C without UV radiation.

Yields obtained from the solution obtained after extraction by dissolving the catalyst: ethanol (10.2 ± 3.0 µmol/g), formic acid (2.4 ± 0.5 µmol/g), acetone (0.9 ± 0.2 µmol/g), acetic acid (0.7 ± 0.2 µmol/g) and methanol (0.6 ± 0.2 µmol/g).

Yields obtained from the liquid water (supernatant): ethanol (7.0 ± 1.1 µmol/g), acetic acid (3.0 ± 1.2 µmol/g), formic acid (1.9 ± 0.8 µmol/g), acetone (1.1 ± 0.4 µmol/g) and methanol (0.2 ± 0.1 µmol/g).

### 9. Synthesis of functionalized organic molecules having 1 to 3 carbon atoms using coated p-HAp as catalyst

Functionalized organic molecules having 1 to 3 carbon atoms were synthesized from CO₂ and CH₄ gas mixture (3 bar each) in the presence of coated p-HAp as catalyst and in the presence of liquid H₂O (1 mL). The reaction was carried out for 72 h at 95 °C and under irradiation of an UV lamp (GPH265T5L/4, 253.7 nm) illuminating directly the coated p-HAp. The p-HA was coated with two layers of aminotris(methylenephosphonic acid) and a layer of zirconium oxychloride (ZrOCl₂), wherein the layer of zirconium oxychloride was arranged or sandwiched between the two layers of aminotris(methylenephosphonic acid). The yields (expressed as µmol of product per gram of coated p-HAp) obtained from the solution obtained after extraction by dissolving the catalyst were: ethanol (16.1 ± 3.2 µmol/g), methanol (4.9 ± 1.0 µmol/g), acetone (0.8 ± 0.2 µmol/g) and acetic acid (0.6 ± 0.1 µmol/g).

### 10. Synthesis of ethanol using coated p-HAp as catalyst

Ethanol was synthesized from CO₂ and CH₄ gas mixture (3 bar each) in the presence of coated p-HAp as catalyst and in the presence of liquid H₂O (1 mL). The reaction was carried out for 72 h at 95 °C and under irradiation of an UV lamp (GPH265T5L/4, 253.7 nm) illuminating directly the coated p-HAp. The p-HA was coated with two layers of aminotris(methylenephosphonic acid) and a layer of zirconium oxychloride (ZrOCl₂), wherein the layer of zirconium oxychloride was arranged or sandwiched between the two layers of aminotris(methylenephosphonic acid). The reaction resulted in the following yields (expressed as µmol of product per gram of coated p-HAp): ethanol (16.1 ± 3.2 µmol/g), methanol (4.9 ± 1.0 µmol/g), malonic acid (1.6 ± 0.2 µmol/g), acetone (0.8 ± 0.2 µmol/g) and acetic acid (0.6 ± 0.1 µmol/g). The predominant product, ethanol, was identified by means of ¹H-NMR spectroscopy not only by the quartet (CH₂) and the triplet (CH₃) at 3.53 ppm and 1.06 ppm, respectively, but also by the intense OH peak at 4.65 ppm.

### 11. Synthesis of ethanol using (uncoated) HAp as catalyst

Ethanol was synthesized from CO₂ and CH₄ gas mixture (3 bar each) in the presence of (uncoated) HAp as catalyst and in the presence of liquid H₂O (1 mL). The reaction was carried out for 72 h at 95 °C and under irradiation of an UV lamp (GPH265T5L/4, 253.7 nm) illuminating directly the p-HAp. The reaction resulted in a very poor yield of ethanol (1.9 ± 0.5 µmol/g catalyst). Further, the yield of acetone and acetic acid was < 0.1 µmol/g catalyst.

### 12. Synthesis of ethanol without a solid support acting as catalyst

Ethanol was synthesized from CO₂ and CH₄ gas mixture (3 bar each) in the presence of (uncoated) HAp as catalyst and in the presence of liquid H₂O (1 mL). The reaction was carried out for 72 h at 95 °C and under irradiation of an UV lamp (GPH265T5L/4, 253.7 nm). In absence of any solid support acting as catalyst (see fig. 7(a)), the yield of ethanol is practically 0 (0.1 ± 0.05 µmol/g). Such a small amount, which has been attributed to eventual photo-induced CO₂ reduction and water splitting, completely disappears in absence of UV radiation (see 7(b)).

### 13. Synthesis of functionalized organic molecules having 1 to 3 carbon atoms, in particular ethanol, using traffic contaminated air

As a proof of concept the reaction for synthesizing ethanol was conducted at atmospheric pressure using contaminated air taken from the surrounding area of the UPC (Universitat Politècnica de Catalunya) east campus in Barcelona, an area heavily contaminated by car traffic, since it is in front of one of the main roads of the city. The contaminated air by combustion of fossil carburant contains significantly higher CO₂ and CH₄ than the average of the ambient air. The reaction was conducted using p-HAp as catalyst, in presence of 1 mL of water and at 95 °C with UV radiation. Analysis of the reaction products after 72 h showed ethanol among other products, some of them non-identified in previous reactions with controlled gas mixtures. Despite the fact that the amount of ethanol (1.1 ± 0.2 µmol/g), acetic acid (0.03 ± 0.01 µmol/g), acetone (0.09 ± 0.02 µmol/g), formic acid (0.13 ± 0.05 µmol/g) and methanol (0.16 ± 0.04 µmol/g) were very small, it was found the formation of high-value chemical products confirming the potential applicability of p-HAp as catalyst to regenerate contaminated air while obtaining functionalized organic molecules having 1 to 3 carbon atoms as valuable products.

### 14. Further investigations in terms of the mechanism pathway

The formation of functionalized organic molecules having 1 to 3 carbon atoms might be associated to the pressure of the feeding gas, the temperature and the reaction time. In order to explore the role of the reaction conditions, the process without UV illumination at was repeated using CO₂ gas and uncoated p-HAp as catalyst. As shown in figs. 12(a)-(d), the yield of functionalized organic molecules having 1 to 3 carbon atoms increases with pressure. The total yield (sum of the yields obtained for each product by dissolving the catalyst + sum of the yields obtained for each product from the supernatant) increased from 11.9 ± 1.6 to 23.1 ± 2.3 µmol/g when the pressure increases from 1 to 6 bars.

In summary, it could be confirmed the catalytic activity of permanently polarized hydroxyapatite to convert gaseous CO₂ in high-value organic chemicals, namely functionalized organic molecules having 1 to 3 carbon atoms, following an electro-reduction mechanism. Experiments under different reaction conditions reflect the formation of functionalized organic molecules having 1 to 3 carbon atoms, which are formed through the permanently polarized hydroxyapatite induced electro-reduction of CO₂. As a proof of concept, the proposed reaction has been successful in obtaining high-value chemical products from road traffic contaminated air, opening an exciting new avenue to transform greenhouse gas emissions into valuable chemical products using a simple catalyst based on an earth-abundant mineral.

## Claims

1. A process for producing functionalized organic molecules having 1 to 3 carbon atoms selected from the group consisting of ethanol, methanol, formic acid, acetic acid, malonic acid, acetone and a mixture of at least two of the afore-said functionalized organic molecules, comprising the step of
- contacting a gas mixture comprising or consisting of carbon dioxide and methane in presence of water with a catalyst comprising or consisting of permanently polarized hydroxyapatite.

2. The process according to claim 1, **characterized in that** the permanently polarized hydroxyapatite has
- a crystallinity > 65 %, in particular > 70 %, preferably > 75 %, more preferably from 65 % to 99 %, and/or
- a proportion of amorphous calcium phosphate < 18 % by weight, in particular from 0.1 % by weight to 17 % by weight, based on the total weight of the permanently polarized hydroxyapatite, and/or
- a proportion of β-tricalcium phosphate < 36 % by weight, in particular from 0.1 % by weight to 35 % by weight, based on the total weight of the permanently polarized hydroxyapatite, and/or
- a bulk resistance from 10⁷ Ω cm² to 10⁵ Ω cm², in particular 10⁶ Ω cm² to 10⁵ Q cm², wherein the bulk resistance preferably increases by only 4 % to 73 %, in particular 4 % to 63 %, preferably by 4%, after 3 months, and/or
- a surface capacitance decreasing less than 8%, in particular from 8 % to 0.1 %, preferably 5 % to 3 %, after 3 months.

3. The process according to claim 1 or 2, **characterized in that** the permanently polarized hydroxyapatite is obtained by a process comprising the steps of
(a) preparing samples of hydroxyapatite,
(b) sintering the samples prepared in step (a) at a temperature between 700 °C and 1200 °C,
(c) applying a constant or variable DC voltage between 250 V and 2500 V, in particular for at least 1 minute at a temperature between 900 °C and 1200 °C, to the samples obtained in step (b) or to shaped bodies thereof or
applying an equivalent electric field between 1.49 kV/cm and 15 kV/cm, in particular for at least 1 minute at a temperature between 900 °C and 1200 °C, to the samples obtained in step (b) or to shaped bodies thereof or
applying an electrostatic discharge between 2500 V and 1500000 V, in particular for less than 10 minutes at a temperature between 900 °C and 1200 °C, to the samples obtained in step (b) or to shaped bodies thereof or
applying an equivalent electric field between 148.9 kV/cm and 8928 kV/cm, in particular for less than 10 minutes at a temperature between 900 °C and 1200 °C, to the samples obtained in step (b) or to shaped bodies thereof and
(d) cooling the samples obtained in step (c) maintaining the DC voltage or the equivalent electric field or
cooling the samples obtained in step (c) maintaining or without maintaining the electrostatic discharge or the equivalent electric field.

4. The process according to any of the preceding claims, **characterized in that** the permanently polarized hydroxyapatite is obtained by a process comprising the steps of
(a) preparing samples of hydroxyapatite,
(b) sintering the samples prepared in step (a) at a temperature of 1000 °C, in particular for 2 h,
(c) applying an equivalent electric field of 3 kV/cm at a temperature of 1000 °C, in particular for 1 h, to the samples obtained in step (b) or to shaped bodies thereof and
(d) cooling the samples obtained in step (c) maintaining the equivalent electric field, in particular for 30 minutes.

5. The process according to any of the preceding claims, **characterized in that** the contacting step is carried out in the presence of liquid water.

6. The process according to any of the preceding claims, **characterized in that** the contacting step is carried out with a volumetric ratio of the permanently polarized hydroxyapatite to water, in particular liquid water, of 1000:1 to 0.01:1, in particular 500:1 to 100:1, preferably 300:1 to 350:1.

7. The process according to any of the preceding claims, **characterized in that** the contacting step is carried out with a volumetric ratio of carbon dioxide to methane of 200:1, in particular 3:1, preferably 1:1.

8. The process according to any of the preceding claims, **characterized in that** the contacting step is carried out under a total pressure of 0.1 bar to 100 bar, in particular 1 bar to 10 bar, preferably of 6 bar.

9. The process according to any of the preceding claims, **characterized in that** the contacting step is carried out under a pressure of carbon dioxide of 0.035 bar to 100 bar, in particular 1 bar to 6 bar, preferably of 6 bar.

10. The process according to any of the preceding claims, **characterized in that** the contacting step is carried out under a partial pressure of carbon dioxide of 0.035 bar to 90 bar, in particular 1 bar to 3 bar, preferably of 3 bar, and/or under a partial pressure of methane of 0.00017 bar to 5 bar, in particular 1 bar to 3 bar, preferably of 3 bar.

11. The process according to any of the preceding claims, **characterized in that** the contacting step is carried out with a molar ratio of carbon dioxide to permanently polarized hydroxyapatite of 0.1 to 0.5, in particular 0.2 to 0.5, preferably 0.3 to 0.5, and/or with a molar ratio of methane to permanently polarized hydroxyapatite of 0.1 to 0.5, in particular 0.2 to 0.5, preferably 0.3 to 0.5.

12. The process according to any of the preceding claims, **characterized in that** the contacting step is carried out under UV irradiation or UV-Vis irradiation having a wavelength from 200 nm to 850 nm, in particular 240 nm to 400 nm, preferably 250 nm to 260 nm, more preferably of 253.7 nm.

13. The process according to any of the preceding claims, **characterized in that** the contacting step is carried out under UV irradiation and or Visible light irradiation, in particular having a irradiance from 0,1 W/m² to 200 W/m², in particular 1 W/m² to 50 W/m², preferably ² W/m² to 10 W/m².

14. The process according to any of the preceding claims, **characterized in that** the contacting step is carried out at a temperature of 25 °C to 250 °C, in particular 95 °C to 140 °C, preferably of 95 °C.

15. Use of a process according to any of the preceding claims for producing ethanol, or a mixture comprising or consisting of ethanol and at least one further functionalized organic molecule selected from the group consisting of methanol, formic acid, acetic acid, malonic acid and acetone, or a mixture comprising or consisting of ethanol, methanol, formic acid, acetic acid and acetone, in particular with ethanol as a major reaction product, or a mixture comprising or consisting of ethanol, methanol, acetic acid, malonic acid and acetone, in particular with ethanol as a major reaction product.

16. Use of a process according to any of claims 1-14 for removing carbon dioxide from the atmosphere.

## Patentansprüche

1. Verfahren zur Herstellung von funktionalisierten organischen Molekülen mit 1 bis 3 Kohlenstoffatomen, die aus der Gruppe bestehend aus Ethanol, Methanol, Ameisensäure, Essigsäure, Malonsäure, Aceton und einem Gemisch von mindestens zwei der obigen funktionalisierten organischen Moleküle ausgewählt sind, umfassend den Schritt
- Inkontaktbringen eines Gasgemischs, das Kohlendioxid und Methan enthält oder daraus besteht, in Gegenwart von Wasser mit einem Katalysator, der permanent polarisierten Hydroxylapatit umfasst oder daraus besteht.

2. Verfahren nach Anspruch 1, **dadurch gekennzeichnet, dass** der permanent polarisierte Hydroxylapatit
- eine Kristallinität > 65 %, insbesondere > 70 %, bevorzugt > 75 %, weiter bevorzugt von 65 % bis 99 %, und/oder
- einen Anteil an amorphem Calciumphosphat < 18 Gew.-%, insbesondere von 0,1 Gew.-% bis 17 Gew.- %, bezogen auf das Gesamtgewicht des permanent polarisierten Hydroxylapatits, und/oder
- einen Anteil an β-Tricalciumphosphat < 36 Gew.-%, insbesondere von 0,1 Gew.-% bis 35 Gew.-%, bezogen auf das Gesamtgewicht des permanent polarisierten Hydroxylapatits, und/oder
- einen Bulkwiderstand von 10⁷ Ω cm² bis 10⁵ Ω cm², insbesondere 10⁶ Ω cm² bis 10⁵ Ω cm², wobei der Bulkwiderstand bevorzugt nach 3 Monaten nur 4 % bis 73 %, insbesondere 4 % bis 63 %, bevorzugt um 4 %, zunimmt, und/oder
- eine Oberflächenkapazität, die nach 3 Monaten um weniger als 8 %, insbesondere von 8 % bis 0,1 %, bevorzugt 5 % bis 3 %, abnimmt,
aufweist.

3. Verfahren nach Anspruch 1 oder 2, **dadurch gekennzeichnet, dass** der permanent polarisierte Hydroxylapatit durch ein Verfahren erhalten wird, das folgende Schritte umfasst:
(a) Herstellen von Proben von Hydroxylapatit,
(b) Sintern der in Schritt (a) hergestellten Proben bei einer Temperatur zwischen 700 °C und 1200 °C,
(c) Anlegen einer konstanten oder variablen Gleichspannung zwischen 250 V und 2500 V, insbesondere über einen Zeitraum von mindestens 1 Minute bei einer Temperatur zwischen 900 °C und 1200 °C, an die in Schritt (b) erhaltenen Proben oder Formkörper davon oder
Anlegen eines äquivalenten elektrischen Felds zwischen 1,49 kV/cm und 15 kV/cm, insbesondere über einen Zeitraum von mindestens 1 Minute bei einer Temperatur zwischen 900 °C und 1200 °C, an die in Schritt (b) erhaltenen Proben oder Formkörper davon oder
Anlegen einer elektrostatischen Entladung zwischen 2500 V und 1.500.000 V, insbesondere über einen Zeitraum von weniger als 10 Minuten bei einer Temperatur zwischen 900 °C und 1200 °C, an die in Schritt (b) erhaltenen Proben oder Formkörper davon oder
Anlegen eines äquivalenten elektrischen Felds zwischen 148,9 kV/cm und 8928 kV/cm, insbesondere über einen Zeitraum von weniger als 10 Minuten bei einer Temperatur zwischen 900 °C und 1200 °C, an die in Schritt (b) erhaltenen Proben oder Formkörper davon und
(d) Abkühlen der in Schritt (c) erhaltenen Proben unter Beibehaltung der Gleichspannung bzw. des äquivalenten elektrischen Felds oder Abkühlen der in Schritt (c) erhaltenen Proben unter Beibehaltung oder ohne Beibehaltung der elektrostatischen Entladung bzw. des äquivalenten elektrischen Felds.

4. Verfahren nach einem der vorhergehenden Ansprüche, **dadurch gekennzeichnet, dass** der permanent polarisierte Hydroxylapatit durch ein Verfahren erhalten wird, das folgende Schritte umfasst:
(a) Herstellen von Proben von Hydroxylapatit,
(b) Sintern der in Schritt (a) hergestellten Proben bei einer Temperatur von 1000 °C, insbesondere über einen Zeitraum von 2 h,
(c) Anlegen eines äquivalenten elektrischen Felds von 3 kV/cm bei einer Temperatur von 1000 °C, insbesondere über einen Zeitraum von 1 h, an die in Schritt (b) erhaltenen Proben oder Formkörper davon und
(d) Abkühlen der in Schritt (c) erhaltenen Proben unter Beibehaltung des äquivalenten elektrischen Felds, insbesondere über einen Zeitraum von 30 Minuten.

5. Verfahren nach einem der vorhergehenden Ansprüche, **dadurch gekennzeichnet, dass** der Schritt des Inkontaktbringens in Gegenwart von flüssigem Wasser durchgeführt wird.

6. Verfahren nach einem der vorhergehenden Ansprüche, **dadurch gekennzeichnet, dass** der Schritt des Inkontaktbringens mit einem volumetrischen Verhältnis von permanent polarisiertem Hydroxylapatit zu Wasser, insbesondere flüssigem Wasser, von 1000:1 bis 0,01:1, insbesondere 500:1 bis 100:1, bevorzugt 300:1 bis 350:1, durchgeführt wird.

7. Verfahren nach einem der vorhergehenden Ansprüche, **dadurch gekennzeichnet, dass** der Schritt des Inkontaktbringens mit einem volumetrischen Verhältnis von Kohlendioxid zu Methan von 200:1, insbesondere 3:1, bevorzugt 1:1, durchgeführt wird.

8. Verfahren nach einem der vorhergehenden Ansprüche, **dadurch gekennzeichnet, dass** der Schritt des Inkontaktbringens unter einem Gesamtdruck von 0,1 bar bis 100 bar, insbesondere 1 bar bis 10 bar, bevorzugt 6 bar, durchgeführt wird.

9. Verfahren nach einem der vorhergehenden Ansprüche, **dadurch gekennzeichnet, dass** der Schritt des Inkontaktbringens unter einem Kohlendioxiddruck von 0,035 bar bis 100 bar, insbesondere 1 bar bis 6 bar, bevorzugt 6 bar, durchgeführt wird.

10. Verfahren nach einem der vorhergehenden Ansprüche, **dadurch gekennzeichnet, dass** der Schritt des Inkontaktbringens unter einem Kohlendioxid-Partialdruck von 0,035 bar bis 90 bar, insbesondere 1 bar bis 3 bar, bevorzugt 3 bar, und/oder unter einem Methan-Partialdruck von 0, 00017 bar bis 5 bar, insbesondere 1 bar bis 3 bar, bevorzugt 3 bar, durchgeführt wird.

11. Verfahren nach einem der vorhergehenden Ansprüche, **dadurch gekennzeichnet, dass** der Schritt des Inkontaktbringens mit einem Molverhältnis von Kohlendioxid zu permanent polarisiertem Hydroxylapatit von 0,1 bis 0,5 insbesondere 0,2 bis 0,5, bevorzugt 0,3 bis 0,5, und/oder mit einem Molverhältnis von Methan zu permanent polarisiertem Hydroxylapatit von 0,1 bis 0,5, insbesondere 0,2 bis 0,5, bevorzugt 0,3 bis 0,5, durchgeführt wird.

12. Verfahren nach einem der vorhergehenden Ansprüche, **dadurch gekennzeichnet, dass** der Schritt des Inkontaktbringens unter UV-Bestrahlung oder UV-Vis-Bestrahlung mit einer Wellenlänge von 200 nm bis 850 nm, insbesondere 240 nm bis 400 nm, bevorzugt 250 nm bis 260 nm, weiter bevorzugt 253,7 nm, durchgeführt wird.

13. Verfahren nach einem der vorhergehenden Ansprüche, **dadurch gekennzeichnet, dass** der Schritt des Inkontaktbringens unter UV-Bestrahlung und/oder Bestrahlung mit sichtbarem Licht, insbesondere mit einer Bestrahlungsstärke von 0,1 W/m² bis 200 W/m², insbesondere 1 W/m² bis 50 W/m², bevorzugt 2 W/m² bis 10 W/m², durchgeführt wird.

14. Verfahren nach einem der vorhergehenden Ansprüche, **dadurch gekennzeichnet, dass** der Schritt des Inkontaktbringens bei einer Temperatur von 25 °C bis 250 °C, insbesondere 95 °C bis 140 °C, bevorzugt 95 °C, durchgeführt wird.

15. Verwendung eines Verfahrens nach einem der vorhergehenden Ansprüche zur Herstellung von Ethanol oder eines Gemischs, das Ethanol und mindestens ein weiteres funktionalisiertes organisches Molekül, das aus der Gruppe bestehend aus Methanol, Ameisensäure, Essigsäure, Malonsäure und Aceton ausgewählt ist, umfasst oder daraus besteht, oder eines Gemischs, das Ethanol, Methanol, Ameisensäure, Essigsäure und Aceton umfasst oder daraus besteht, insbesondere mit Ethanol als Reaktionshauptprodukt, oder eines Gemischs, das Ethanol, Methanol, Essigsäure, Malonsäure und Aceton umfasst oder daraus besteht, insbesondere mit Ethanol als Reaktionshauptprodukt.

16. Verwendung eines Verfahrens nach einem der Ansprüche 1-14 zur Entfernung von Kohlendioxid aus der Atmosphäre.

## Revendications

1. Procédé pour la production de molécules organiques fonctionnalisées possédant 1 à 3 atomes de carbone choisies dans le groupe constitué par l'éthanol, le méthanol, l'acide formique, l'acide acétique, l'acide malonique, l'acétone et un mélange d'au moins deux des molécules organiques fonctionnalisées mentionnées précédemment, comprenant l'étape de
- mise en contact d'un mélange de gaz comprenant ou constitué de dioxyde de carbone et de méthane en présence d'eau avec un catalyseur comprenant ou constitué d'hydroxyapatite polarisée de manière permanente.

2. Procédé selon la revendication 1, **caractérisé en ce que** l'hydroxyapatite polarisée de manière permanente possède
- une cristallinité > 65 %, en particulier > 70 %, préférablement > 75 %, plus préférablement de 65 % à 99 %, et/ou
- une proportion de phosphate de calcium amorphe < 18 % en poids, en particulier de 0,1 % en poids à 17 % en poids, sur la base du poids total de l'hydroxyapatite polarisée de manière permanente, et/ou
- une proportion de phosphate β-tricalcique < 36 % en poids, en particulier de 0,1 % en poids à 35 % en poids, sur la base du poids total de l'hydroxyapatite polarisée de manière permanente, et/ou
- une résistance volumique de 10⁷ Ω cm² à 10⁵ Ω cm², en particulier de 10⁶ Ω cm² à 10⁵ Ω cm², la résistance volumique augmentant préférablement de seulement 4 % à 73 %, en particulier de 4 % à 63 %, préférablement de 4 %, après 3 mois, et/ou
- une capacité de surface diminuant de moins de 8 %, en particulier de 8 % à 0,1 %, préférablement de 5 % à 3 %, après 3 mois.

3. Procédé selon la revendication 1 ou 2, **caractérisé en ce que** l'hydroxyapatite polarisée de manière permanente est obtenue par un procédé comprenant les étapes de
(a) préparation d'échantillons d'hydroxyapatite,
(b) frittage des échantillons préparés dans l'étape (a) à une température comprise entre 700 °C et 1 200 °C,
(c) application d'une tension CC constante ou variable comprise entre 250 V et 2 500 V, en particulier pendant au moins 1 minute à une température comprise entre 900 °C et 1 200 °C, aux échantillons obtenus dans l'étape (b) ou aux corps façonnés correspondants ou
application d'un champ électrique équivalent compris entre 1,49 kV/cm et 15 kV/cm, en particulier pendant au moins 1 minute à une température comprise entre 900 °C et 1 200 °C, aux échantillons obtenus dans l'étape (b) ou aux corps façonnés correspondants ou
application d'une décharge électrostatique comprise entre 2 500 V et 1 500 000 V, en particulier pendant moins de 10 minutes à une température comprise entre 900 °C et 1 200 °C, aux échantillons obtenus dans l'étape (b) ou aux corps façonnés correspondants ou
application d'un champ électrique équivalent compris entre 148,9 kV/cm et 8 928 kV/cm, en particulier pendant moins de 10 minutes à une température comprise entre 900 °C et 1 200 °C, aux échantillons obtenus dans l'étape (b) ou aux corps façonnés correspondants et
(d) refroidissement des échantillons obtenus dans l'étape (c) en maintenant la tension CC ou le champ électrique équivalent ou
refroidissement des échantillons obtenus dans l'étape (c) en maintenant ou sans maintenir la décharge électrostatique ou le champ électrique équivalent.

4. Procédé selon l'une quelconque des revendications précédentes, **caractérisé en ce que** l'hydroxyapatite polarisée de manière permanente est obtenue par un procédé comprenant les étapes de
(a) préparation d'échantillons d'hydroxyapatite,
(b) frittage des échantillons préparés dans l'étape (a) à une température de 1 000 °C, en particulier pendant 2 h,
(c) application d'un champ électrique équivalent de 3 kV/cm à une température de 1 000 °C, en particulier pendant 1 h, aux échantillons obtenus dans l'étape (b) ou aux corps façonnés correspondants et
(d) refroidissement des échantillons obtenus dans l'étape (c) en maintenant le champ électrique équivalent, en particulier pendant 30 minutes.

5. Procédé selon l'une quelconque des revendications précédentes, **caractérisé en ce que** l'étape de mise en contact est réalisée en présence d'eau liquide.

6. Procédé selon l'une quelconque des revendications précédentes, **caractérisé en ce que** l'étape de mise en contact est réalisée avec un rapport volumétrique de l'hydroxyapatite polarisée de manière permanente sur l'eau, en particulier l'eau liquide, de 1 000 : 1 à 0,01 : 1, en particulier de 500 : 1 à 100 : 1, préférablement de 300 : 1 à 350 : 1.

7. Procédé selon l'une quelconque des revendications précédentes, **caractérisé en ce que** l'étape de mise en contact est réalisée avec un rapport volumétrique de dioxyde de carbone sur méthane de 200 : 1, en particulier de 3 : 1, préférablement de 1 : 1.

8. Procédé selon l'une quelconque des revendications précédentes, **caractérisé en ce que** l'étape de mise en contact est réalisée sous une pression totale de 0,1 bar à 100 bars, en particulier de 1 bar à 10 bars, préférablement de 6 bars.

9. Procédé selon l'une quelconque des revendications précédentes, **caractérisé en ce que** l'étape de mise en contact est réalisée sous une pression de dioxyde de carbone de 0,035 bar à 100 bars, en particulier de 1 bar à 6 bars, préférablement de 6 bars.

10. Procédé selon l'une quelconque des revendications précédentes, **caractérisé en ce que** l'étape de mise en contact est réalisée sous une pression partielle de dioxyde de carbone de 0,035 bar à 90 bars, en particulier de 1 bar à 3 bars, préférablement de 3 bars, et/ou sous une pression partielle de méthane de 0,00017 bar à 5 bars, en particulier de 1 bar à 3 bars, préférablement de 3 bars.

11. Procédé selon l'une quelconque des revendications précédentes, **caractérisé en ce que** l'étape de mise en contact est réalisée avec un rapport molaire de dioxyde de carbone sur hydroxyapatite polarisée de manière permanente de 0,1 à 0,5, en particulier de 0,2 à 0,5, préférablement de 0,3 à 0,5, et/ou avec un rapport molaire de méthane sur hydroxyapatite polarisée de manière permanente de 0,1 à 0,5, en particulier de 0,2 à 0,5, préférablement de 0,3 à 0,5.

12. Procédé selon l'une quelconque des revendications précédentes, **caractérisé en ce que** l'étape de mise en contact est réalisée sous irradiation UV ou irradiation UV-Vis possédant une longueur d'onde de 200 nm à 850 nm, en particulier de 240 nm à 400 nm, préférablement de 250 nm à 260 nm, plus préférablement de 253,7 nm.

13. Procédé selon l'une quelconque des revendications précédentes, **caractérisé en ce que** l'étape de mise en contact est réalisée sous irradiation UV et/ou irradiation de la lumière visible, en particulier possédant une irradiance de 0,1 W/m² à 200 W/m², en particulier de 1 W/m² à 50 W/m², préférablement de 2 W/m² à 10 W/m².

14. Procédé selon l'une quelconque des revendications précédentes, **caractérisé en ce que** l'étape de mise en contact est réalisée à une température de 25 °C à 250 °C, en particulier de 95 °C à 140 °C, préférablement de 95 °C.

15. Utilisation d'un procédé selon l'une quelconque des revendications précédentes pour la production d'éthanol, ou d'un mélange comprenant ou constitué d'éthanol et d'au moins une molécule organique fonctionnalisée supplémentaire choisie dans le groupe constitué par le méthanol, l'acide formique, l'acide acétique, l'acide malonique et l'acétone, ou d'un mélange comprenant ou constitué d'éthanol, de méthanol, d'acide formique, d'acide acétique et d'acétone, en particulier avec l'éthanol comme produit de réaction majoritaire, ou d'un mélange comprenant ou constitué d'éthanol, de méthanol, d'acide acétique, d'acide malonique et d'acétone, en particulier avec l'éthanol comme produit de réaction majoritaire.

16. Utilisation d'un procédé selon l'une quelconque des revendications 1 à 14 pour l'élimination de dioxyde de carbone de l'atmosphère.
